(19) Europäisches Patentamt
European Patent Office
Office européen des brevets

(11) Veröffentlichungsnummer: **0 013 424**
**B1**

(12) # EUROPÄISCHE PATENTSCHRIFT

(45) Veröffentlichungstag der Patentschrift: **17.11.82**

(21) Anmeldenummer: **79105374.7**

(22) Anmeldetag: **27.12.79**

(51) Int. Cl.³: **C 07 D 495/10,** A 61 K 31/55 // C07D211/64, C07C149/40, C07C149/36, C07C149/34, C07C149/415 ,(C07D495/10, 337/00, 221/00)

(54) **Spiro(dibenz(b,f)thiepin-piperidine), Zwischenprodukte zu ihrer Herstellung und sie enthaltende Arzneimittel.**

(30) Priorität: **10.01.79 US 2350**

(43) Veröffentlichungstag der Anmeldung: **23.07.80 Patentblatt 80/15**

(45) Bekanntmachung des Hinweises auf die Patenterteilung: **17.11.82 Patentblatt 82/46**

(84) Benannte Vertragsstaaten: **AT BE CH DE FR GB IT NL**

(56) Entgegenhaltungen: **DE-A-2 504 642**

(73) Patentinhaber: **HOECHST AKTIENGESELLSCHAFT, Postfach 80 03 20, D-6230 Frankfurt/Main 80 (DE)**

(72) Erfinder: **Ong, Helen Hu, Bunker Hill Place 15 Whippany, New Jersey (US)**
Erfinder: **Profitt, James Arthur, Barclay Place 1805 Goshen, Indiana 46526 (US)**

EP 0 013 424 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Spiro[dibenz(b,f)thiepin-piperidine], Zwischenprodukte zu ihrer Herstellung und sie enthaltende Arzneimittel

Vorliegende Erfindung betrifft neue Spiro[dibenz(b,f)thiepin-piperidine] sowie deren pharmazeutisch unbedenklichen Säureadditionssalze, welche als Analgetika, Tranquilizer und Anticonvulsia nützlich sind, und pharmazeutische Zusammensetzungen, die eine solche Verbindung als wesentlichen Wirkstoff enthalten. Die erfindungsgemäßen Verbindungen entsprechen der Formel I

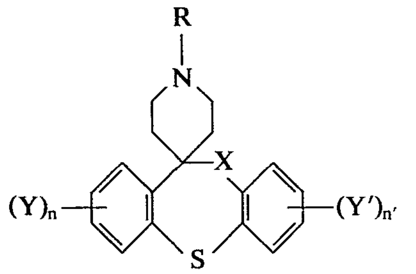

(I)

worin R für Wasserstoff, $C_1-C_6$ Alkyl, $C_1-C_6$ Hydroxyalkyl, $C_3-C_7$-Cycloalkyl-$C_1-C_6$-alkyl, Phenylalkyl, Phenoxyalkyl oder Benzoylalkyl, worin die Alkylgruppe jeweils 1—6 C-Atome besitzt und der Phenylkern jeweils durch Nitro, Amino, Chlor, Fluor, Brom, Methoxy, $C_1-C_6$-Alkyl oder Trifluormethyl einfach oder mehrfach substituiert sein kann, $C_2-C_6$-Alkanoyl, Cyan oder ein Äthylenglykolketal der Formel

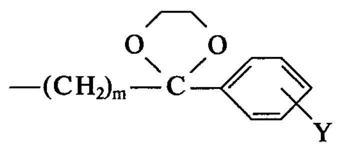

oder einen Rest der Formel

$$—CH_2—R^1$$

mit $R^1$ $C_2$–$C_5$-Alkenyl oder $C_2$–$C_5$-Alkinyl steht, X

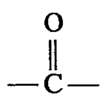

darstellt, wenn R die oben angegebene Bedeutung hat, oder

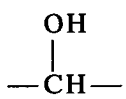

darstellt, wenn R für $C_1-C_6$-Alkyl, Wasserstoff oder Cyan steht, Y und Y′ gleich oder verschieden sind und je Wasserstoff, Chlor, Fluor, Brom, Methoxy, Methylthio oder Trifluormethyl bedeuten können, n und n′ gleich oder verschieden sind und je eine ganze Zahl von 1 bis einschließlich 2 darstellen können und m eine ganze Zahl von 1 bis einschließlich 4 ist.

Die vorliegende Erfindung betrifft ferner Verbindungen der Formel I, worin Y, Y′, n und n′ die oben angegebene Bedeutung haben, X die Carbonylgruppe und R die Cyangruppe bedeutet, oder X die Gruppe

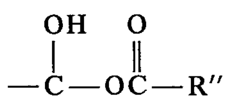

darstellt und R für

$—CO—R'$ $\quad CH_3$ oder $CN$

steht, wobei R′ und R″ gleich oder verschieden sind und jeweils $C_1-C_6$-Alkyl bedeuten, als Zwischenprodukte zur Herstellung von Verbindungen der Formel I.

Die zur Herstellung der erfindungsgemäßen, pharmazeutisch unbedenklichen Säuresalze verwendbaren Säuren umfassen anorganische Säuren wie Salzsäure, Bromwasserstoffsäure,

Schwefelsäure, Salpetersäure, Phosphorsäure und Überchlorsäure sowie organische Säuren wie Weinsäure, Zitronensäure, Essigsäure, Bernsteinsäure, Maleinsäure, Fumarsäure und Oxalsäure.

Die Verbindungen der vorliegenden Erfindung wurden bisher noch nicht beschrieben. Galt u. a., in der U.S. 4 001 419, beschrieben als Analgetika 1'-substituierte Xanthen-9-spiro-4'-piperidinderivate der Formel

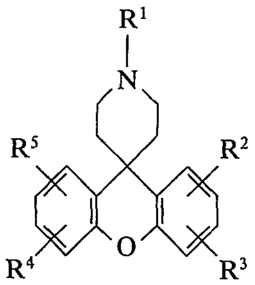

worin $R^1$ für ein Wasserstoffatom, einen Alkylrest mit 1 bis 10 Kohlenstoffatomen, einen Alkenylrest mit 3 bis 10 Kohlenstoffatomen, einen Halogenalkenylrest mit 3 bis 6 Kohlenstoffatomen, einen gegebenenfalls im Cycloalkylkern durch einen Arylrest mit 6 bis 10 Kohlenstoffatomen oder ein oder zwei Alkylreste mit 1 bis 3 Kohlenstoffatomen substituierten Cycloalkylalkylrest mit 4 bis 7 Kohlenstoffatomen, einen Phenylrest, einen gegebenenfalls im Arylkern durch eins bis drei Halogenatome oder Alkylreste mit 1 bis 3 Kohlenstoffatomen substituierten Arylalkylrest mit 7 bis 10 Kohlenstoffatomen, einen Hydroxyalkylrest mit 2 bis 5 Kohlenstoffatomen, einen Dialkylaminoalkylrest mit 4 bis 8 Kohlenstoffatomen, einen Carbamoylalkylrest mit 2 bis 8 Kohlenstoffatomen, einen Alkylcarbamoylalkylrest mit 3 bis 8 Kohlenstoffatomen oder einen Alkanoylalkylrest mit 3 bis 8 Kohlenstoffatomen steht, $R^2$, $R^3$, $R^4$ und $R^5$ gleich oder verschieden sind und je Wasserstoffatome, Halogenatome, Alkylreste mit 1 bis 5 Kohlenstoffatomen, Halogenalkylreste mit 1 bis 5 Kohlenstoffatomen, Alkoxyreste mit 1 bis 5 Kohlenstoffatomen, Alkylthioreste mit 1 bis 5 Kohlenstoffatomen, Hydroxylreste, Thiolreste, Alkanoylaminoreste mit 1 bis 5 Kohlenstoffatomen, Alkanoyloxyreste mit 1 bis 5 Kohlenstoffatomen, gegebenenfalls im Arylkern durch eins bis drei Halogenatome oder Alkylreste mit 1 bis 3 Kohlenstoffatomen substituierte Aroyloxyreste mit 7 bis 10 Kohlenstoffatomen, Arylalkenyloxyreste mit 9 bis 12 Kohlenstoffatomen, Hydroxyalkylreste mit 1 bis 5 Kohlenstoffatomen, Alkylsulfinylreste mit 1 bis 5 Kohlenstoffatomen oder Alkansulfonylreste mit 1 bis 5 Kohlenstoffatomen bedeuten können, sowie deren pharmazeutisch unbedenkliche Säureadditionssalze.

Ferner offenbaren Gerecke u. a., in der französischen Patentschrift 824 066, als Tranquilizer tricyclische Verbindungen der Formel

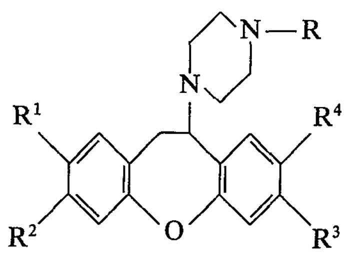

worin R eine Niederalkyl-, Niederalkenylalkyl- oder Niederalkinylalkylgruppe bedeutet, welche im Fall, wo sie von einer Methylgruppe verschieden ist, durch eine Cyan-Hydroxyl- oder Alkanoyloxygruppe substituiert sein kann, oder worin R eine Gruppe der allgemeinen Formel

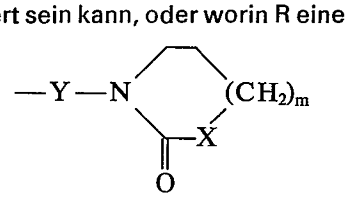

in der X ein Sauerstoff- oder Schwefelatom, eine Iminogruppe, eine Niederalkyliminogruppe oder eine Methylengruppe und Y eine gegebenenfalls durch eine Niederalkylgruppe substituierte Äthylen- oder Propylengruppe darstellen und m gleich 0 oder 1 ist, bedeutet und ferner eine der Substituenten $R_1$ und $R_2$ für ein Wasserstoffatom und der andere für eine Niederalkyl-, Niederalkoxy- oder Niederalkylthiogruppe, ein Halogen oder eine Trifluormethyl- oder Hydroxylgruppe steht sowie einer der Substituenten $R_3$ und $R_4$ für Wasserstoff und der andere für ein Wasserstoffatom, eine Niederalkyl-, Niederalkoxy- oder Niederalkylthiogruppe, ein Halogen oder eine Trifluormethyl- oder

Hydroxylgruppe steht, sowie Salze dieser Verbindungen.

Die erfindungsgemäßen Verbindungen lassen sich nach einer oder mehreren der folgenden Methoden herstellen, wobei R, X, Y, Y' und Z die oben definierte Bedeutung haben, falls nicht anders angegeben.

Methode A

1. Man unterwirft ein 2-Phenylthiophenylacetonitril der Formel II

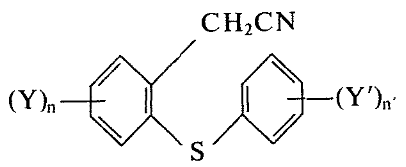

(II)

einer Bisalkylierung mit Mechloräthaminhydrochlorid in Gegenwart einer starken Base (als säurebindendes Mittel) bei einer Temperatur im Bereich von etwa Raumtemperatur bis etwa 85° C, was das entsprechende 4-Cyan-1-methyl-4-(2-phenylthiophenyl)-piperidin der Formel III

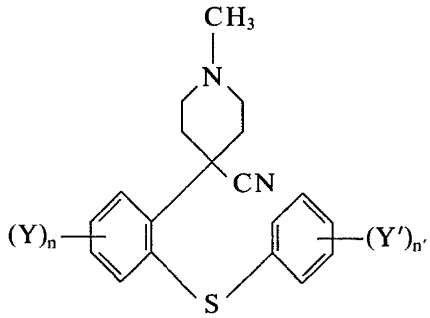

(III)

liefert. Bevozugte Arbeitsweisen schließen die Verwendung von Dimethylformamid oder Dimethylsulfoxid als Lösungsmittel und von Natriumhydrid als säurebindendes Mittel ein.

2. Eine Verbindung der Formel III wird gemäß der ersten Stufe der von Braunreaktion behandelt, was das entsprechende 1,4-Dicyan-4-(2-phenylthiophenyl)-piperidin der Formel IV

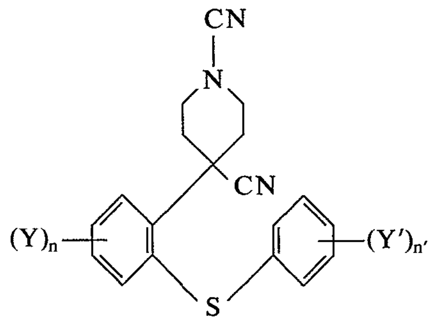

(IV)

liefert. Bei dieser Umsetzung wird Bromcyan zum Ersatz des N-Methyls eingesetzt. Die Reaktionsbedingungen umfassen ferner ein Lösungsmittel wie Chloroform oder Methylenchlorid, eine als säurebindendes Mittel wirkende milde Base wie Kaliumcarbonat sowie eine Reaktionstemperatur im Bereich von etwa Raumtemperatur bis zum Rückfluß des Reaktionsgemischs.

3. Man unterwirft eine Verbindung der Formel IV einer sauren Hydrolyse, was die entsprechende 4-(2-Phenylthiophenyl)-piperidin-4-carbonsäure der Formel V

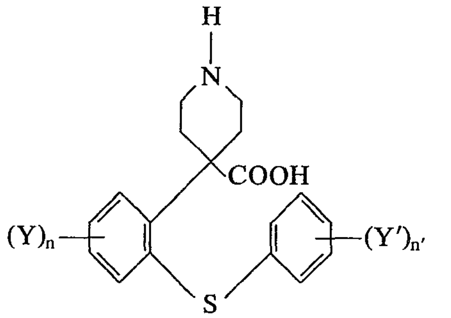

(V)

liefert. Bei einer bevorzugten Methode wird konzentrierte (48%) Bromwasserstoffsäure unter Rückflußbedingungen eingesetzt.

4. Eine Verbindung der Formel V wird in Gegenwart eines basischen Lösungsmittels oder eines säurebindenden Mittels acyliert, was die entsprechende 1-Alkanoyl-4-(2-phenylthiophenyl)-piperidin-4-carbonsäure der Formel

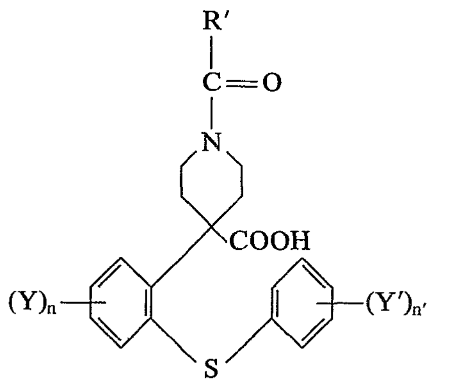

(VI)

worin R′ für Alkyl steht, liefert. Vorzugsweise ist das basische Lösungsmittel Pyridin und das säurebindende Mittel Kaliumcarbonat. Das Acylierungsmittel kann ein geeignetes Alkanoylhalogenid, z. B. Acetylchlorid, oder ein Anhydrid, z. B. Essigsäureanhydrid, sein.

5. Eine Verbindung der Formel VI setzt man vorsichtig mit einem Thionylhalogenid um, was das entsprechende Säurehalogenid der Formel VII

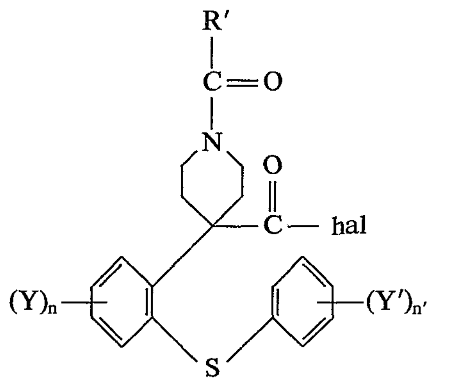

(VII)

liefert. Bei einer bevorzugten Arbeitsweise wird Thionylchlorid eingesetzt und die Reaktion durch etwa 5 Minuten Erhitzen auf dem Dampfbad durchgeführt.

6. Eine Verbindung der Formel VII wird cyclisiert, was das entsprechende 10,11-Dihydro-1'-alkanoyl-11-oxospiro[dibenz[b,f]thiepin-10,4'-piperidin], eine Verbindung der Formel Ia

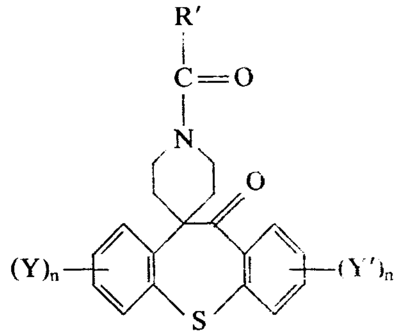

(Ia)

liefert. Diese Cyclisierung läßt sich über das Säurechlorid unter modifizierten Friedel-Crafts-Bedingungen mit einem Lewissäurekatalysator wie Aluminiumchlorid oder Eisen-III-chlorid durchführen. Methylenchlorid wird als Lösungsmittel bevorzugt. Die Reaktionstemperatur kann zwischen etwa 15°C und etwa 150°C liegen. Bei einer bevorzugten Arbeitsweise werden das Säurechlorid der Formel VII, Aluminiumchlorid, Methylenchlorid und Rückflußbedingungen angewandt.

Methode B

1. Man unterwirft eine Verbindung der Formel III einer sauren Hydrolyse nach Methode A (3), was die entsprechende Carbonsäure der Formel

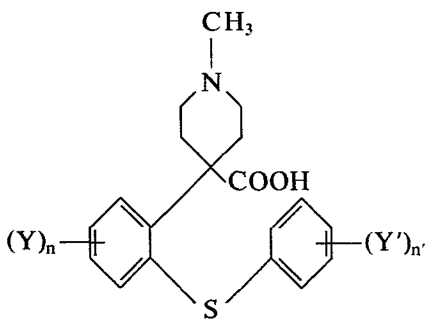

(VIII)

liefert.

2. Eine Verbindung der Formel VIII wird cyclisiert, was das entsprechende 10,11-Dihydro-1'-methyl-11-oxospiro[dibenz(b,f)thiepin-10,4'-piperidin], eine Verbindung der Formel Ib

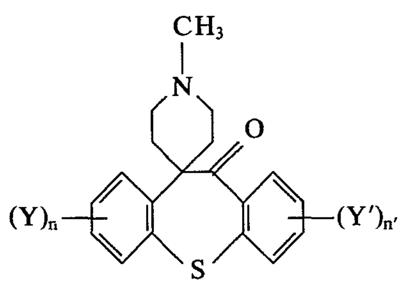

(Ib)

liefert. Bei einer bevorzugten Arbeitsweise verwendet man Polyphosphorsäure und eine Reaktionstemperatur von etwa 95—115°C. Andernfalls läßt sich die Arbeitsweise der Methode A (6) anwenden.

Methode C

Man unterwirft eine Verbindung der Formel Ia einer sauren Hydrolyse, was die entsprechende erfindungsgemäße N-unsubstituierte Verbindung der Formel

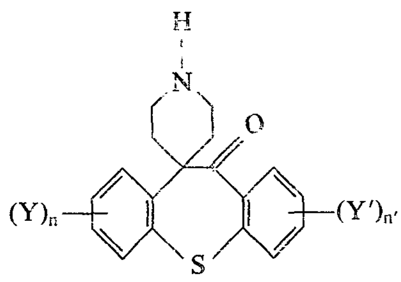

(Ic)

liefert. 3n-Salzsäure unter Rückflußbedingungen stellt eine bevorzugte Arbeitsweise zur Durchführung dieser Hydrolyse dar.

Methode D

1. Man behandelt eine Verbindung der Formel Ib bzw. If weiter unten gemäß der Methode A (2), was die entsprechende erfindungsgemäße N-Cyanverbindung der Formel

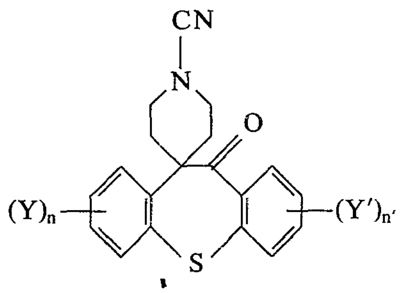

(Id)

liefert.

2. Eine Verbindung der Formel Id wird einer Hydrolyse gemäß der zweiten Stufe der von Braunreaktion unterworfen, was die entsprechende erfindungsgemäße N-unsubstituierte Verbindung der obigen Formel Ic liefert. Bei dieser Arbeitsweise werden ein Gemisch aus 3n-Salzsäure und Eisessig (2 : 1) sowie Temperaturen im Bereich von etwa 75 bis 150°C angewandt.

Methode E

Eine Verbindung der obigen Formeln Ia, Ib oder Id läßt sich unter Friedel-Carftsbedingungen acylieren, wie in Methode A (6) beschrieben, was die entsprechende erfindungsgemäße 11-Alkanoyloxy-11-hydroxyverbindung der Formel

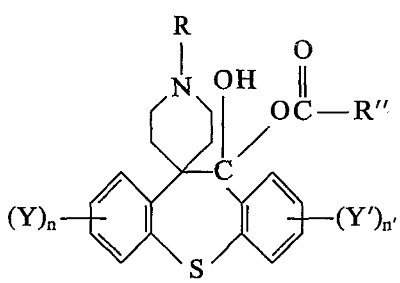

(Ie)

worin R für

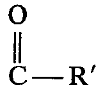 $CH_3$ oder CN

steht sowie R′ und R″ gleich oder verschieden sind und je Alkyl bedeuten, liefert.

Methode F

Eine Verbindung der obigen Formeln Ia, Ib, Ic oder Id wird reduziert, was die entsprechende erfindungsgemäße 11-Hydroxyverbindung der Formel

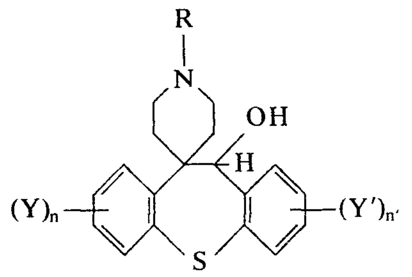

(If)

worin R für Alkyl oder H steht, liefert. Bei einer bevorzugten Arbeitsweise verwendet man Lithiumaluminiumhydrid als Reduktionsmittel, Tetrahydrofuran als Lösungsmittel und eine Reaktionstemperatur im Bereich von Raumtemperatur bis zum Rückfluß des Reaktionsgemischs.

Methode G

Eine Verbindung der Formel Ic oder If (R=H) wird nach irgendeiner geeigneten, dem Stand der Technik bekannten Methode mit einem entsprechenden Reagenz alkyliert bzw. acyliert, was die entsprechende erfindungsgemäße N-substituierte Verbindung der Formel

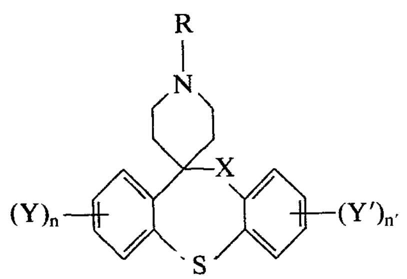

(Ig)

worin R für Alkyl, Hydroxyalkyl, Alkinyl, Alkenyl, Phenalkyl, Phenoxyalkyl, Cycloalkylalkyl, Alkanoyl oder Benzoylalkyl und X für

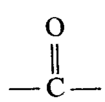

oder

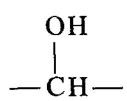

stehen, liefert. Bei bevorzugten Arbeitsweisen setzt man ein Alkylierungsmittel der Formel R-Halogenid ein, wobei die Alkylierung in einem Lösungsmittel wie Dimethylformamid, mit einem säurebindenden Mittel wie Natriumbicarbonat oder Kaliumcarbonat, einem Reaktionsinitiator wie Kaliumjodid und bei einer Reaktionstemperatur im Bereich von etwa Raumtemperatur bis zum Rückfluß des Reaktionsgemischs erfolgt.

Methode H

1. Eine Verbindung der Formel X oder XIII (R=H) wird unter Bedingungen, die mit denen der Methode G übereinstimmen, mit einem Chloräthylenglykolketal der Formel

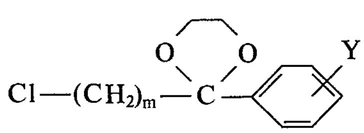

behandelt, was die entsprechende erfindungsgemäße Verbindung, worin R ein Benzoylalkyläthylenketal darstellt, liefert.

2. Man unterwirft eine erfindungsgemäße Äthylenketalverbindung einer sauren Hydrolyse, was die entsprechende erfindungsgemäße Verbindung, worin R Benzoylniederalkyl

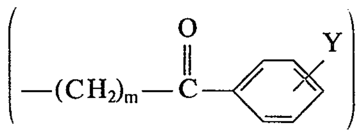

darstellt, liefert. Bei einer bevorzugten Arbeitweise verwendet man 3n-Salzsäure in Äthanol als das die Hydrolyse bewirkende Mittel.

Methode I

Man behandelt eine Verbindung der Formel Ic oder If (R=H) gemäß den Bedingungen der Eschweiler-Clarkereaktion, was die entsprechende erfindungsgemäße Verbindung, worin R Methyl darstellt, liefert. Diese Methode wird durch Umsetzung mit Ameisensäure und Formaldehyd unter Rückflußbedingungen durchgeführt.

Bei allen obigen Methoden sind die optimalen Bedingungen von den Ausgangsstoffen, Lösungsmitteln, Katalysatoren und sonstigen Reaktionsteilnehmern abhängig. Dies wird in den nachfolgenden Beispielen weiter erläutert.

Durch die Formel I dargestellte Ausgangsstoffe sind entweder im Handel erhältlich oder nach wohlbekannten Methoden gemäß dem folgenden Reaktionsschema herstellbar:

1.

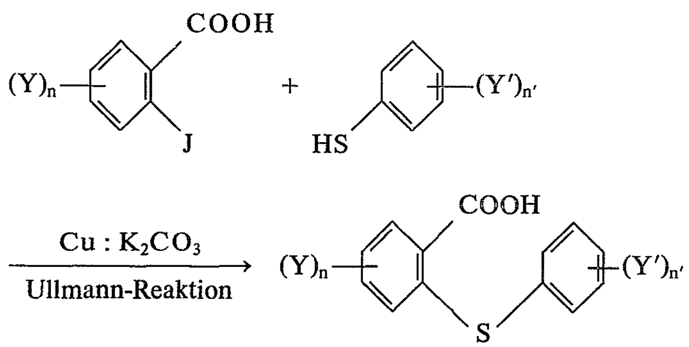


2.

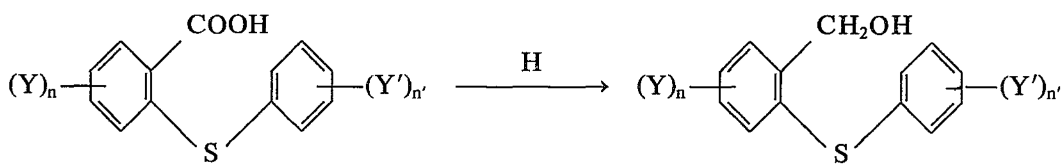


Reduktion mit Natrium-bis-(2-methoxyäthoxy)-aluminiumhydrid (VITRIDE®)

3.

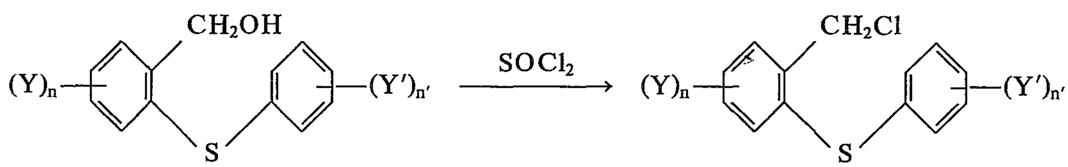


4.

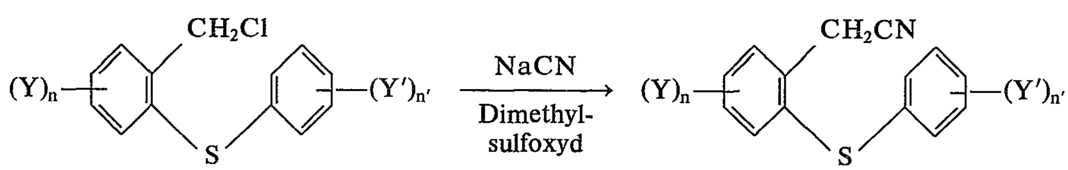


(I)

Die erfindungsgemäßen Verbindungen sind wegen ihrer Fähigkeit bei Säugetieren Schmerzen zu lindern, als Analgetika wertvoll, wie beim durch Phenyl-α-chinon ausgelösten Krümmungssyndrom an der Maus, einem Standardtest auf Analgesie [Proc. Soc. Exptl. Biol. Med. 95, 729 (1957)], nachgewiesen.

Erfindungsgemäße Verbindungen sind ebenso bei Verabreichung in Tagesmengen im Bereich von etwa 0,1 bis etwa 100 mg/kg Körpergewicht zur Linderung von Schmerzen in Säugetieren verwendbar.

Verbindungen der vorliegenden Erfindung sind ferner als Tranquilizer wertvoll, wie durch ihre Fähigkeit, Aggressivität nach Pfotenschock zu hemmen, nachgewiesen [Arch. Int. Pharmacodynam. et de Therap. 142, 30 (1963)]. Erfindungsgemäße Verbindungen sind bei Verabreichung in Tagesmengen im Bereich von etwa 0,5 bis etwa 100 mg/kg Körpergewicht in Säugetieren als Tranquilizer verwendbar.

Verbindungen der vorliegenden Erfindung sind ferner als Krampfmittel für Säugetiere wertvoll, wie nach L. A. Woodbury und V. D. Davenport [Arch. Int. Pharmacodynam. 92, S. 97 – 107 (1952)] bestimmt. Erfindungsgemäße Verbindungen sind bei Verabreichung in Tagesmengen im Bereich von etwa 0,5 bis 100 mg/kg Körpergewicht zur Behandlung von Krämpfen in Säugetieren verwendbar.

Erfindungsgemäße Verbindungen sind unter anderem:

8-Chlor-10,11-dihydro-2-methoxy-1′-methyl-11-oxospiro-[dibenz(b,f)thiepin-10,4′-piperidin],
3-Brom-10,11-dihydro-7-methylthio-1′-methyl-11-oxospiro-[dibenz(b,f)thiepin-10,4′-piperidin],
10,11-Dihydro-3-methoxy-1′-methyl-11-oxo-8-trifluormethylspiro-[dibenz(b,f)thiepin-10,4′-piperidin],
10,11-Dihydro-9-methoxy-1′-methyl-2-methyl-11-oxospiro-[dibenz(b,f)thiepin-10,4′-piperidin],
10,11-Dihydro-1′-methyl-8-methyl-11-oxo-2-trifluormethylspiro-[dibenz(b,f)thiepin-10,4′-piperidin],
2-Brom-7-chlor-10,11-dihydro-1′-methyl-11-oxospiro-[dibenz(b,f)thiepin-10,4′-piperidin],
10,11-Dihydro-2,8-dimethoxy-1′-methyl-11-oxospiro-[dibenz(b,f)thiepin-10,4′-piperidin],
10,11-Dihydro-1′-methyl-2-methylthio-11-oxo-7-trifluormethylspiro-[dibenz(b,f)thiepin-10,4′-piperidin],
2-Fluor-1′-[2-(p-fluorbenzoyl)-äthyl]-10,11-dihydro-11-oxospiro-[dibenz(b,f)thiepin-10,4′-piperidin],
2-Chlor-1′-[2-(p-fluorbenzoyl)-äthyl]-10,11-dihydro-11-oxospiro-[dibenz(b,f)thiepin-10,4′-piperidin],
1′-(4-Benzoylbutyl)-10,11-dihydro-11-oxospiro[dibenz(b,f)thiepin-10,4′-piperidin],
1′-(4-Benzoylbutyl)-2-chlor-10,11-dihydro-11-oxospiro-[dibenz(b,f)thiepin-10,4′-piperidin],
1′-(4-Benzoylbutyl)-2-fluor-10,11-dihydro-11-oxospiro-[dibenz(b,f)thiepin-10,4′-piperidin],
2-Fluor-10,11-dihydro-7,8-dimethoxy-1′-methyl-11-oxospiro-[dibenz(b,f)thiepin-10,4′-piperidin],
2,3-Dichlor-10,11-dihydro-1′-methyl-7-methylthio-11-oxospiro-[dibenz(b,f)thiepin-10,4′-piperidin],
8-Chlor-2,3-difluor-10,11-dihydro-1′-methyl-11-oxospiro-[dibenz(b,f)thiepin-10,4′-piperidin] und
2-Chlor-7,8-difluor-10,11-dihydro-1′-methyl-11-oxospiro-[dibenz(b,f)thiepin-10,4′-piperidin].

Wirksame Mengen der erfindungsgemäßen Verbindungen können einem Patienten nach irgendeiner von verschiedenen Methoden verabreicht werden, beispielsweise peroral wie in Kapseln oder Tabletten, parenteral in Form steriler Lösungen oder Suspensionen sowie in einigen Fällen intravenös in Form steriler Lösungen. Die Endprodukte selbst sind zwar als freie Base wirksam, doch kann man sie aus Gründen der Stabilität, Leichtigkeit der Kristallisation, erhöhter Löslichkeit und dergleichen in Form ihrer pharmazeutisch unbedenklichen Additionssalze formulieren und verabreichen.

Die erfindungsgemäßen Wirkstoffe lassen sich peroral verabreichen, beispielsweise mit einem inerten Streckmittel oder eßbaren Träger, oder sie können in Gelatinekapseln eingeschlossen oder zu Tabletten komprimiert werden. Zur peroralen therapeutischen Verabreichung kann man die erfindungsgemäßen Wirkstoffe in Vehikel einarbeiten und in Form von Tabletten, Pastillen, Kapseln, Elixieren, Suspensionen, Sirupen, Oblaten, Kaugummi und dergleichen anwenden. Diese Präparate sollen mindestens 0,5% Wirkstoff enthalten, aber dies läßt sich in Abhängigkeit von der jeweiligen Form variieren und kann zweckmäßig zwischen 4% und etwa 70% des Einheitsgewichts liegen. Eine solche Wirkstoffmenge liegt in derartigen Zusammensetzungen vor, daß sich eine geeignete Dosis ergibt. Bevorzugte Zusammensetzungen und Präparate gemäß vorliegender Erfindung werden so zubereitet, daß eine perorale Dosiereinheitsform 1,0 bis 300 Milligramm Wirkstoff enthält.

Die Tabletten, Pillen, Kapseln, Pastillen und dergleichen können ferner folgende Bestandteile

enthalten: ein Bindemittel, wie mikrokristalline Cellulose, Traganthgummi oder Gelatine; ein Vehikel wie Stärke oder Milchzucker, ein Sprengmittel wie Alginsäure, Primogel, Maisstärke und dergleichen; ein Schmiermittel wie Magnesiumstearat oder Sterotex, ein Gleitmittel wie kolloidales Siliciumdioxyd; ferner kann man einen Süßstoff wie Rohrzucker oder Saccharin oder auch einen Geschmacksstoff wie Pfefferminz, Methylsalicylat oder Orangenaroma zusetzen. Ist die Dosiereinheitsform eine Kapsel, so kann diese neben Stoffen der obigen Art einen flüssigen Träger wie ein fettes Öl enthalten. Andere Dosiereinheitsformen können weitere verschiedene Stoffe enthalten, welche die physikalische Form der Dosiereinheit modifizieren, wie beispielsweise Überzüge. Tabletten oder Pillen können somit mit Zucker oder Schellack überzogen oder mit anderen, erst im Darm löslichen Überzügen versehen werden. Ein Sirup kann neben den Wirkstoffen Rohrzucker als Süßstoff sowie gewisse Konservierungsmittel, Farbstoffe und Farben sowie Geschmacksstoffe enthalten. Bei der Bereitung dieser verschiedenen Zusammensetzungen verwendete Stoffe sollen pharmazeutisch rein und in den verwendeten Mengen nicht toxisch sein.

Zur parenteralen therapeutischen Verabreichung können die erfindungsgemäßen Wirkstoffe in eine Lösung oder Suspension eingearbeitet werden. Diese Zubereitungen sollen mindestens 0,1% Wirkstoff enthalten, doch läßt sich dies zwischen 0,5 und etwa 50% ihres Gewichts variieren. Eine solche Wirkstoffmenge liegt in derartigen Zusammensetzungen vor, daß sich eine geeignete Dosis ergibt. Bevorzugte Zusammensetzungen und Präparate gemäß vorliegender Erfindung werden so hergestellt, daß eine parenterale Dosiereinheit 0,5 bis 100 Milligramm Wirkstoff enthält.

Die Lösungen oder Suspensionen können ferner folgende Bestandteile umfassen: ein steriles Verdünnungsmittel wie Wasser zur Injektion, physiologische Kochsalzlösung, nichtflüchtige Öle, Polyäthylenglykole, Propylenglykol oder sonstige synthetische Lösungsmittel; antibakterielle Mittel wie Benzylalkohol oder Methylparaben; Antioxydantien wie Ascorbinsäure oder Natriumbisulfit; Chelatbildner wie Äthylendiamintetraessigsäure; Puffer wie Acetate, Citrate oder Phosphate sowie Mittel zur Einstellung des osmotischen Drucks, wie Kochsalz oder Dextrose. Das parenterale Präparat kann in Ampullen, zum einmaligen Gebrauch bestimmten Spritzen oder Mehrfachdosis-Phiolen aus Glas oder Kunststoff eingeschmolzen werden.

## Beispiel 1

a) Unter Stickstoff gibt man im Verlauf von 5 Minuten 2,8 g 99%iges Natriumhydrid portionsweise zu einer Lösung von 6,0 g 2-(4-Chlorphenylthio)-benzylcyanid in 36 ml Dimethylsulfoxyd. Nach beendeter Zugabe wird 4 Minuten gerührt, bevor man portionsweise im Verlauf von 35 Minuten eine Lösung von 4,7 g 95%igem Mechloräthaminhydrochlorid in 24 ml Dimethylsulfoxyd zusetzt. Danach erhitzt man 80 Minuten auf 70–80° C und läßt dann auf Raumtemperatur abkühlen. Das abgekühlte Gemisch wird auf 175 ml Eis gegossen und das wäßrige Gemisch dreimal mit je 75 ml Äther extrahiert. Die vereinigten Ätherextrakte wäscht man dreimal mit je 50 ml Wasser und extrahiert dann mit 40 ml 2n-Salzsäure. Das weiße Salz wird durch Filtrieren gewonnen, nacheinander mit Wasser und Äther gewaschen und getrocknet. Der Feststoff wird aus Aceton und dann zweimal aus Methylalkohol/Äther umkristallisiert, wobei man 4-[2-(4-Chlorphenylthio)-phenyl]-4-cyan-1-methylpiperidinhydrochlorid als Produkt vom Schmelzpunkt 257–259° C erhält.

b) Man rührt ein Gemisch aus 6,3 g 4-[2-(4-Chlorphenylthio)-phenyl]-4-cyan-1-methylpiperidinhydrochlorid und 26 ml 48%iger Bromwasserstoffsäure 60 Stunden in einem Bad bei 135–140° C und versetzt dann mit 13 ml Eisessig. Unter fortgesetztem Rühren hält man 24 Stunden bei dieser Temperatur. Danach wird mit 250 ml Wasser verdünnt und das verdünnte Gemisch in einem Bad von 90° C am Rotationsverdampfer eingedampft. Diesen Verdünnungs- und Eindampfungsvorgang wiederholt man zweimal, nimmt den Endrückstand in 50 ml Wasser auf und gibt dann 5 ml 58%ige Ammoniumhydroxydlösung dazu, wobei ein weißes Pulver ausfällt. Das Pulver wird isoliert, mit Aceton gewaschen und aus Methylalkohol umkristallisiert, wobei man 4-[2-(4-Chlorphenylthio)-phenyl]-1-methylpiperidin-4-carbonsäure als reines Produkt vom Schmelzpunkt 284–285° C erhält.

c) Man rührt ein Gemisch aus 4,4 g 4-[2-(4-Chlorphenylthio)-phenyl]-1-methylpiperidin-4-carbonsäure und 40 ml Polyphosphorsäure unter Stickstoff 2 Stunden bei 100–110° C. Danach kühlt man auf 0° C ab und versetzt mit 10 ml Eis und 70 ml Wasser. Das Gemisch wird mit konzentrierter Ammoniumhydroxydlösung alkalisch gemacht und viermal mit je ml Äther extrahiert. Die vereinigten Ätherextrakte wäscht man nacheinander mit 100 ml Wasser, 100 ml 10%iger Natronlauge und 50 ml gesättigter Kochsalzlösung. Nach dem Waschen wird die Lösung zur Trockne gebracht. Nach Entfernung des Lösungsmittels wird die Rohbase auf einer Silikagel-60-Säule mit Methylalkohol/Chloroform (1 : 6) als Eluiermittel chromatographiert. Die Base überführt man in Äther in ihr Hydrobromidsalz, 2-Chlor-10,11-dihydro-1'-methyl-11-oxospiro[dibenz(b,f)thiepin-10,4'-piperidin-hydrobromid].

### Beispiel 2

a) Man gibt 2,6 g 99%iges Natriumhydrid portionsweise zu einer Lösung von 4,4 g 2-Phenylthiophenylacetonitril in 50 ml Dimethylformamid. Das dabei gebildete Gemisch wird 30 Minuten gerührt, bevor man eine Lösung von 4,0 g Mechloräthaminhydrochlorid in 50 ml Dimethylformamid dazutropft, wobei man die Gasentwicklungsgeschwindigkeit niedrig hält. Nach beendeter Zugabe wird das Gemisch 15 Stunden bei 80 – 85°C gerührt. Danach gibt man 100 g Eis dazu, bevor das Gemisch dreimal mit Äther extrahiert wird. Die vereinigten Ätherextrakte trocknet man über Kaliumcarbonat und entfernt dann das Lösungsmittel im Vakuum. Den Rückstand löst man in einer kleinen Menge Äther und läßt ihn durch eine in Äther gefüllte Aluminiumoxydsäule laufen. Beim Eluieren mit Äther erhält man ein schweres Öl. Dieses wird in sein Hydrochlorid, 4-Cyan-1-methyl-4-(2-phenylthiophenyl)-piperidinhydrochlorid, überführt, welches man aus Aceton/Äther umkristallisiert.

b) Unter Rühren gibt man eine Lösung von 2,6 g 4-Cyan-1-methyl-4-(2-phenylthiophenyl)-piperidin, der freien Base aus a), in 22 ml Chloroform rasch zu einem Gemisch aus 1,5 g Bromcyan und 6 g Kaliumcarbonat in 30 ml Chloroform. Das Reaktionsgemisch wird 16 Stunden unter Rückfluß gerührt und danach filtriert. Das Filtrat wird im Vakuum eingeengt.

Den Rückstand löst man in einigen ml Methylalkohol, um gegebenenfalls nicht umgesetztes Bromcyan zu zersetzen. Der Methylalkohol wird dann abgedampft, wobei ein blaßgelblicher Rückstand verbleibt, den man aus Aceton umkristallisiert, wobei man 1,4-Dicyan-4-(2-phenylthiophenyl)-piperidin erhält.

c) Man rührt eine Suspension von 8 g 1,4-Dicyan-4-(2-phenylthiophenyl)-piperidin in 120 ml 48%iger Bromwasserstoffsäure 16 Stunden unter Rückfluß. Überschüssige Säure wird dann durch Vakuumdestillation entfernt. Den Rückstand löst man in 100 ml Wasser. Beim Abkühlen trübt sich die Lösung, und Kristalle beginnen sich abzuscheiden. Das Gemisch wird 16 Stunden lang bei 0°C gehalten und dann filtriert, wobei man 4-(2-Phenylthiophenyl)-piperidin-4-carbonsäurehydrobromid erhält.

d) Man erhitzt ein Gemisch aus 8 g 4-(2-Phenylthiophenyl)-piperidin-4-carbonsäurehydrobromid und 10 ml Essigsäureanhydrid in 50 ml Pyridin 4 Stunden zum Rückfluß. Überschüssiges Pyridin wird dann durch Vakuumdestillation entfernt und der Rückstand mit 100 ml 1n-Salzsäure angerieben und dann dreimal mit Chloroform extrahiert. Die vereinigten Chloroformextrakte werden nacheinander mit Wasser gut gewaschen, getrocknet und im Vakuum eingeengt. Der Rückstand wird aus siedendem Aceton umkristallisiert, wobei man 1-Acetyl-4-(2-phenylthiophenyl)-piperidin-4-carbonsäure erhält.

e) Man erhitzt eine Suspension von 3,4 g 1-Acetyl-4-(2-phenylthiophenyl)-piperidin-4-carbonsäure in 5 ml frisch destilliertem Thionylchlorid 5 Minuten auf dem Dampfbad, wobei sich eine klare Lösung ergibt. Überschüssiges Thionylchlorid wird unter vermindertem Druck bei 50°C entfernt. Der Rückstand wird aus Benzol/Cyclohexan umkristallisiert, wobei man 1-Acetyl-4-(2-phenylthiophenyl)-piperidin-4-carbonylchlorid erhält.

f) Man gibt eine Lösung von 14 g 1-Acetyl-4-(2-phenylthiophenyl)-piperidin-4-carbonylchlorid in 500 ml Methylenchlorid langsam unter Rühren zu 7,5 g Aluminiumchlorid. Nach beendeter Zugabe erhitzt man 2 Stunden zum Rückfluß. Anschließend wird Eis zugesetzt, bevor man dreimal mit Chloroform extrahiert. Die vereinigten Chloroformextrakte werden nacheinander mit verdünntem Alkali und mit Wasser gewasche, getrocknet und im Vakuum vom Lösungsmittel befreit, wobei man 10,11-Dihydro-1′-acetyl-11-oxospiro[dibenz(b,f)thiepin-10,4′-piperidin] erhält.

### Beispiel 3

Man rührt eine Suspension von 2,0 g 10,11-Dihydro-1′-acetyl-11-oxospiro[dibenz(b,f)thiepin-10,4′-piperidin], Beispiel 2, in 15 ml 3n-Salzsäure 3 Stunden unter Rückfluß. Die entstandene Lösung wird mit kaltem Wasser verdünnt und dann mit Essigester extrahiert. Die saure wäßrige Lösung macht man mit Kaliumcarbonat alkalisch, wobei ein Amin freigesetzt wird, das man in Äther auflöst. Die Ätherlösung wird nacheinander getrocknet und im Vakuum eingeengt, wobei ein Öl verbleibt, welches man in Äther in sein Bromwasserstoffsäuresalz, 10,11-Dihydro-11-oxospiro[dibenz(b,f)thiepin-10,4′-piperidin]-hydrobromid, überführt.

### Beispiel 4

Man erhitzt ein Gemisch aus 3,5 g 2-Chlor-10,11-dihydro-1′-methyl-11-oxospiro[dibenz(b,f)thiepin-10,4′-piperidin], der freien Base aus Beispiel 1, 7,1 g Kaliumcarbonat und 1,8 g Bromcyan in 45 ml Methylenchlorid 2 Stunden unter Rückfluß. Dann filtriert man und dampft das Filtrat am Rotationsverdampfer ein. Der Rückstand wird in 30 ml Methylalkohol aufgelöst und die Lösung 10 Minuten am Rückfluß erhitzt und dann wiederum am Rotationsverdampfer eingedampft. Den Rückstand chromatographiert man mit Äther über 50 g Silikagel 60. Die verbleibende Substanz wird in Chloroform gelöst und die Chloroformlösung am Rotationsverdampfer eingedampft, wobei

2-Chlor-1'-cyan-10,11-dihydro-11-oxospiro[dibenz(b,f)thiepin-10,4'-piperidin] vom Schmelzpunkt 141,5 – 142° C verbleibt.

Beispiel 5

Man rührt eine homogene Lösung von 1,8 g 2-Chlor-1'-cyan-10,11-dihydro-11-oxospiro[dibenz(b,f)thiepin-10,4'-piperidin], Beispiel 4, in 20 ml 3n-Salzsäure und 10 ml Eisessig 16 Stunden bei 110 – 120° C und läßt dann auf Raumtemperatur abkühlen. Die Lösung wird anschließend mit 50 ml Wasser verdünnt und dann bei 80° C am Rotationsverdampfer bis zur Entfernung von 50 ml Wasser eingedampft, worauf man den Verdünnungs- und Verdampfungsschritt wiederholt. Der Rückstand wird mit 200 ml Wasser verdünnt und die verdünnte Lösung nacheinander im Eisbad gekühlt, mit einer kleinen Menge 58%igem Ammoniumhydroxyd auf pH 9 basisch gemacht und dreimal mit je 80 ml Äther extrahiert. Die vereinigten Ätherextrakte wäscht man nacheinander zweimal mit je 80 ml Wasser und einmal mit 40 ml gesättigtem Kochsalz, trocknet dann über Magnesiumsulfat und dampft schließlich zur Trockne ein. Den Rückstand löst man in Äther und überführt ihn in sein Bromwasserstoffsäuresalz, 2-Chlor-10,11-dihydro-11-oxospiro[dibenz(b,f)thiepin-10,4'-piperidin]-hydrobromid.

Beispiel 6

Im Verlauf von 1 – 2 Minuten gibt man eine Lösung von 0,4 g Acetylchlorid in Methylenchlorid unter Rühren zu einem Gemisch aus 1,0 g 1'-Acetyl-10,11-dihydro-11-oxospiro[dibenz(b,f)thiepin-10,4'-piperidin], Beispiel 2, und 0,94 g Aluminiumchlorid in 20 ml Methylenchlorid. Nach beendeter Zugabe rührt man 16 Stunden bei Raumtemperatur und zersetzt dann mit Eiswasser. Anschließend wird die organische Phase dreimal mit Essigester/Äther (1 : 1) extrahiert, nacheinander mit Natriumbicarbonatlösung, Wasser und Kochsalzlösung gewaschen, getrocknet und im Vakuum eingeengt, wobei man 10,11-Dihydro-11-acetoxy-1'-acetyl-11-hydroxyspiro[dibenz(b,f)thiepin-10,4'-piperidin] erhält.

Beispiel 7

Man tropft eine Lösung von 2,4 g 10,11-Dihydro-11-oxospiro[dibenz(b,f)thiepin-10,4'-piperidin], der freien Base aus Beispiel 3, in 20 ml Tetrahydrofuran zu einer zum Rückfluß erhitzten Aufschlämmung von 1,5 g Lithiumaluminiumhydrid in 20 ml Tetrahydrofuran. Nach beendeter Zugabe rührt man noch weitere 4 Stunden unter Rückfluß und dann 16 Stunden bei Raumtemperatur. Anschließend wird das Reaktionsgemisch durch aufeinanderfolgende Zugabe von 1,7 ml Wasser mit 1,7 ml 15%iger Natronlauge und 5,2 ml Wasser zersetzt. Das verdünnte Gemisch wird filtriert und das Filtrat im Vakuum eingeengt, wobei man 10,11-Dihydro-11-hydroxyspiro[dibenz(b,f)thiepin-10,4'-piperidin] erhält.

Beispiel 8

Man erhitzt ein Gemisch aus 1,1 g 10,11-Dihydro-11-oxospiro[dibenz(b,f)thiepin-10,4'-piperidin], der freien Base aus Beispiel 3, in 8 ml Ameisensäure 2 Stunden zum Rückfluß und setzt dann 7,2 ml Formalin (38% Formaldehyd in Wasser) dazu. Nach der Zugabe wird 16 Stunden weitergerührt und am Rückfluß erhitzt. Anschließend wird das Reagenzgemisch bei vermindertem Druck entfernt und der Rückstand mit verdünntem Alkali basisch gemacht. Ein Öl scheidet sich ab und wird mit Äther extrahiert. Man trocknet die vereinigten Ätherextrakte und dampft dann im Vakuum ein. Das Öl wird in Äther gelöst und in sein Bromwasserstoffsäuresalz, 10,11-Dihydro-1'-methyl-11-oxospiro[dibenz(b,f)thiepin-10,4'-piperidin]-hydrobromid, überführt.

Beispiel 9

Man behandelt eine Lösung von 5,0 g 10,11-Dihydro-1'-methyl-11-oxospiro[dibenz(b,f)thiepin-10,4'-piperidin], der freien Base aus Beispiel 8, in 40 ml Tetrahydrofuran gemäß der Arbeitsweise von Beispiel 7, wobei man 10,11-Dihydro-11-hydroxy-1'-methylspiro[dibenz(b,f)thiepin-10,4'-piperidin] erhält.

Beispiel 10

Man rührt ein Gemisch aus 1,0 g 10,11-Dihydro-11-oxospiro[dibenz(b,f)thiepin-10,4'-piperidin], der freien Base aus Beispiel 3, 0,3 g Propargylchlorid und 1 g Natriumbicarbonat in 15 ml Dimethylformamid 16 Stunden bei 70° C. Danach wird das Gemisch nacheinander mit Äther verdünnt, filtriert, mit Wasser gewaschen, getrocknet und im Vakuum eingeengt. Den Rückstand überführt man in Äther in sein Bromwasserstoffsäuresalz, 10,11-Dihydro-11-oxo-1'-(2-propinyl)-spiro[dibenz(b,f)thiepin-10,4'-piperidin]-hydrobromid

Beispiel 11

Man rührt ein Gemisch aus 1,1 g 10,11-Dihydro-11-oxospiro[dibenz(b,f)-10,4'-piperidin], der freien Base aus Beispiel 3, 0,63 g $\beta$-Bromäthylalkohol und 1,1 g Natriumbicarbonat in 15 ml Dimethylformamid 16 Stunden bei 80° C. Dann verdünnt man mit Äther und Wasser. Die Ätherlösung wird mit einem Überschuß 2n-Salzsäure extrahiert. Den sauren Extrakt macht man mit Kaliumcarbonat basisch. Das abgeschiedene Öl wird in Äther gelöst, getrocknet und zur Trockne eingeengt. Die so erhaltene ölige Base wird in Äther in ihr Bromwasserstoffsäuresalz, 10,11-Dihydro-1'-($\beta$-hydroxyäthyl)-11-oxospiro[dibenz(b,f)thiepin-10,4'-piperidin]-hydrobromid, überführt.

Beispiel 12

Man rührt ein Gemisch aus 2,0 g 10,11-Dihydro-11-oxospiro[dibenz(b,f)thiepin-10,4'-piperidin], der freien Base aus Beispiel 3, 0,74 g Chlormethylcyclopropan, 1,8 g Natriumbicarbonat und 1,8 g Kaliumjodid in 15 ml Dimethylformamid 16 Stunden bei 70–80° C. Man läßt die entstandene Suspension abkühlen und verdünnt dann mit Äther und Wasser. Die organische Phase wird nacheinander getrocknet, filtriert und im Vakuum eingeengt. Den Rückstand löst man in Chloroform und läßt die Chloroformlösung mit Äther als Eluiermittel durch eine Aluminiumoxydsäule laufen. Das gereinigte Produkt wird in Äther gelöst und in sein Bromwasserstoffsäuresalz, 1'-Cyclopropylmethyl-10,11-dihydro-11-oxospiro[dibenz(b,f)thiepin-10,4'-piperidin]-hydrobromid, überführt.

Beispiele 13 und 14

Man behandelt Proben von 10,11-Dihydro-11-oxospiro[dibenz(b,f)thiepin-10,4'-piperidin], der freien Base aus Beispiel 3, nach der Arbeitsweise von Beispiel 12 getrennt mit Phenoxypropylbromid bzw. 2-Bromäthylbenzol, wobei man (Beispiel 13) 10,11-Dihydro-11-oxo-1'-(3-phenoxypropyl)-spiro[dibenz(b,f)thiepin-10,4'-piperidin]-hydrobromid bzw. (Beispiel 14) 10,11-Dihydro-1'-phenäthyl-11-oxospiro[dibenz(b,f)thiepin-10,4'-piperidin]-hydrobromid erhält.

Beispiel 15

Man rührt ein Gemisch aus 1,3 g 10,11-Dihydro-11-oxospiro[dibenz(b,f)thiepin-10,4'-piperidin], der freien Base aus Beispiel 3, 1,4 g $\gamma$-Chlor-4-fluorbutyrophenon-äthylenglykolketal, 1,0 g Natriumbicarbonat und 1,0 g Kaliumjodid in 15 ml Dimethylformamid 16 Stunden bei 80° C. Anschließend wird das Reaktionsgemisch filtriert und das Filtrat eingeengt. Der Rückstand wird in durch Vermischen von 16 ml absolutem Äthylalkohol mit 6 ml 3n-Salzsäure hergestellter äthanolischer Salzsäure gelöst und 16 Stunden bei Raumtemperatur gerührt. Die saure Lösung macht man mit 40%iger Natronlauge alkalisch, wobei ein Amin frei wird, das mit Äther extrahiert wird. Man trocknet die vereinigten Ätherextrakte und engt dann zur Trockne ein. Den Rückstand läßt man mit Äther als Eluiermittel durch eine Aluminiumoxydsäule laufen. Das gereinigte Produkt wird in Äther in sein Bromwasserstoffsäuresalz, 1'-[3-(4-Fluorbenzoyl)-propyl]-10,11-dihydro-11-oxospiro[dibenz(b,f)thiepin-10,4'-piperidin]-hydrobromid, überführt.

Beispiel 16

Man rührt ein Gemisch aus 2,5 g 2-Chlor-10,11-dihydro-11-oxospiro[dibenz(b,f)thiepin-10,4'-piperidin], der freien Base aus Beispiel 5, 2,5 g Natriumbicarbonat, 2,0 g Kaliumjodid und 0,8 g Chlormethylcyclopropan in 30 ml Dimethylformamid 16 Stunden bei 80° C. Anschließend kühlt man im Eisbad und verdünnt dann mit 90 ml Wasser. Die wäßrige Phase wird zweimal mit je 40 ml Äther extrahiert, mit 58%igem Ammoniumhydroxyd auf pH 9 gestellt und erneut mit zweimal je 40 ml Äther extrahiert. Die vereinigten Ätherextrakte wäscht man nacheinander zweimal mit je 40 ml Wasser und

einmal mit 20 ml gesättigter Kochsalzlösung und trocknet. Die getrocknete Lösung läßt man mit Äther als Eluiermittel durch eine Aluminiumoxydsäule laufen. Das gereinigte Produkt löst man in Äther und überführt es in sein Bromwasserstoffsäuresalz, 2-Chlor-1'-cyclopropylmethyl-10,11-dihydro-11-oxo[dibenz(b,f)thiepin-10,4'-piperidin]-hydrobromid.

Beispiel 17

Man behandelt ein Gemisch aus 2,5 g 2-Chlor-10,11-dihydro-11-oxospiro[dibenz(b,f)thiepin-10,4'-piperidin], der freien Base aus Beispiel 5, 2,5 g Natriumbicarbonat, 2,0 g Kaliumjodid und 1,1 g Propargylbromid in 30 ml Dimethylformamid gemäß der Arbeitsweise von Beispiel 16, wobei man 2-Chlor-10,11-dihydro-11-oxo-1'-(2-propinyl)-spiro[dibenz(b,f)thiepin-10,4'-piperidin] erhält.

Beispiel 18

0,9 g Allylbromid in Dimethylformamid gibt man portionsweise im Verlauf von 60 Minuten zu einem 65° C warmen Gemisch aus 2,1 g 2-Chlor-10,11-dihydro-11-oxospiro[dibenz(b,f)thiepin-10,4'-piperidin], der freien Base aus Beispiel 5, und 2,1 g Natriumbicarbonat in 15 ml Dimethylformamid. Nach beendeter Zugabe rührt man 16 Stunden bei 60—65° C. Anschließend wird mit 75 ml Wasser verdünnt und das zweiphasige Gemisch mit 40 ml Äther extrahiert. Die wäßrige Schicht und ein sich gegebenenfalls an der Grenzfläche bildender Niederschlag werden dreimal mit je 40 ml Methylenchlorid extrahiert. Die vereinigten organischen Phasen wäscht man nacheinander zweimal mit je 50 ml Wasser und mit 25 ml gesättigter Kochsalzlösung und trocknet. Die Lösung läßt man mit Äther als Eluiermittel durch eine Aluminiumoxydsäule laufen. Das gereinigte Produkt wird in Äther gelöst und in sein Bromwasserstoffsäuresalz, 1'-Allyl-2-chlor-10,11-dihydro-11-oxospiro[dibenz(b,f)thiepin-10,4'-piperidin]-hydrobromid, überführt.

Beispiel 19

Ein Gemisch aus 1,2 g Äthyljodid und 10 ml Dimethylformamid gibt man portionsweise im Verlauf von 30 Minuten zu einem 80°C warmen Gemisch aus 2,1 g 2-Chlor-10,11-dihydro-11-oxospiro[dibenz(b,f)thiepin-10,4'-piperidin], der freien Base aus Beispiel 5, und 2,5 g $NaHCO_3$ in 15 ml Dimethylformamid. Nach beendeter Zugabe kühlt man das Reaktionsgemisch auf 0° C, verdünnt dann mit 75 ml Wasser und extrahiert danach mit 40 ml Äther. Beide Phasen werden durch Papier hindurch filtriert, und der Filterkuchen wird in 75 ml Chloroform gelöst. Man wäscht die Chloroformlösung mit Wasser und bringt dann zur Trockne. Den Rückstand läßt man mit Äther als Eluiermittel durch eine Aluminiumoxydsäule laufen. Das gereinigte Produkt wird in Äther in sein Bromwasserstoffsäuresalz, 2-Chlor-10,11-dihydro-1'-äthyl-11-oxospiro[dibenz(d,f)thiepin-10,4'-piperidin]-hydrobromid, überführt.

Beispiel 20

Man rührt ein Gemisch aus 2,1 g 2-Chlor-10,11-dihydro-11-oxospiro[dibenz(b,f)thiepin-10,4'-piperidin], der freien Base aus Beispiel 5, und 2,1 g Natriumbicarbonat in 15 ml Dimethylformamid bei 85°C und gibt dann im Verlauf von 30 Minuten portionsweise ein Gemisch aus 1,3 g Jodpropan und 10 ml Dimethylformamid dazu. Nach beendeter Zugabe wird der Ansatz 16 Stunden bei 80—85° C gerührt und dann 48 Stunden bei Raumtemperatur stehengelassen. Danach verdünnt man mit 75 ml Wasser und extrahiert dreimal mit je 40 ml Äther. Die vereinigten Ätherextrakte werden nacheinander zweimal mit je 30 ml Wasser und mit 20 ml gesättigter Kochsalzlösung gewaschen und zur Trockne gebracht. Den so erhaltenen Rückstand löst man in einem Gemisch aus Äthylalkohol und 3n-Salzsäure (2 : 1). Die saure Lösung wird mit 58%iger Ammoniumhydroxydlösung auf pH 9 basisch gemacht und dann viermal mit je 40 ml Äther extrahiert. Den Extrakt läßt man mit Äther als Eluiermittel durch eine Aluminiumoxydsäule laufen. Das gereinigte Produkt löst man in Äther und überführt es in sein Bromwasserstoffsäuresalz, 2-Chlor-10,11-dihydro-11-oxo-1'-propylspiro[dibenz(b,f)thiepin-10,4'-piperidin]-hydrobromid.

Beispiel 21

Ein Gemisch aus 1,0 g 1-Brom-3-methyl-2-buten und 10 ml Dimethylformamid gibt man portionsweise im Verlauf von 20 Minuten unter Rühren zu einem 80°C warmen Gemisch aus 1,9 g 2-Chlor-10,11-dihydro-11-oxospiro[dibenz(b,f)thiepin-10,4'-piperidin], der freien Base aus Beispiel 5,

und 1,0 g Natriumbicarbonat in 15 ml Dimethylformamid. Nach beendeter Zugabe rührt man 16 Stunden bei 80° C. Danach wird mit 75 ml Wasser verdünnt und das verdünnte Gemisch dreimal mit je 40 ml Äther extrahiert. Die vereinigten Ätherextrakte wäscht man nacheinander zweimal mit je 30 ml Wasser und einmal mit 20 ml gesättigter Kochsalzlösung und bringt zur Trockne. Den so erhaltenen Rückstand löst man in Äther und überführt ihn in sein Bromwasserstoffsäuresalze, 2-Chlor-10,11-dihydro-1'-(2-methyl-2-butenyl)-11-oxospiro[dibenz(b,f)thiepin-10,4'-piperidin]-hydrobromid.

Beispiel 22

Eine Lösung von 0,93 g 2-Bromäthylalkohol in 10 ml Dimethylformamid gibt man portionsweise im Verlauf von 30 Minuten unter Rühren zu einem 80° C warmen Gemisch aus 2,1 g 2-Chlor-10,11-dihydro-11-oxospiro[dibenz(b,f)thiepin-10,4'-piperidin], der freien Base aus Beispiel 5, 2,1 g Natriumbicarbonat und 1,7 g Kaliumjodid in 15 ml Dimethylformamid. Nach beendeter Zugabe rührt man 16 Stunden bei 80—85° C. Danach wird mit 75 ml Wasser verdünnt und das verdünnte Gemisch dreimal mit je 40 ml Äther extrahiert. Die vereinigten Ätherextrakte wäscht man nacheinander zweimal mit je 30 ml Wasser und extrahiert zweimal mit je 36 ml 2n-Salzsäure. Das vereinigte saure Gemisch wird abgekühlt und dann mit 58%igem Ammoniumhydroxyd auf pH 9 basisch gemacht. Das basische Gemisch extrahiert man dreimal mit je 40 ml Äther, wäscht anschließend die vereinigten Ätherextrakte einmal mit 40 ml Wasser und bringt zur Trockne. Den Rückstand läßt man mit Methylalkohol/Äther (1 : 1) als Eluiermittel durch eine Aluminiumoxydsäule laufen. Das gereinigte Produkt wird in Äther gelöst und in sein Bromwasserstoffsäuresalz, 2-Chlor-10,11-dihydro-1'-(2-hydroxyäthyl)-11-oxospiro[dibenz(b,f)thiepin-10,4'-piperidin]-hydrobromid, überführt.

Beispiel 23

Man rührt ein Gemisch aus 2,1 g 2-Chlor-10,11-dihydro-11-oxospiro[dibenz(b,f)thiepin-10,4'-piperidin], der freien Base aus Beispiel 5, 2,1 g Natriumbicarbonat, 1,7 g Kaliumjodid und 1,4 g 2-Bromäthylbenzol in 25 ml Dimethylformamid 18 Stunden bei 50—65° C. Danach wird mit 75 ml Wasser verdünnt und das verdünnte Gemisch dreimal mit je 40 ml Äther extrahiert. Die vereinigten Ätherextrakte wäscht man nacheinander zweimal mit je 30 ml Wasser und einmal mit 20 ml gesättigter Kochsalzlösung und bringt zur Trockne. Den Rückstand läßt man mit Äther als Eluiermittel durch eine Aluminiumoxydsäule laufen. Der gereingte Rückstand wird mit Hexan behandelt und das Hexangemisch abgekühlt und filtriert, wobei man 2-Chlor-10,11-dihydro-11-oxo-1'-($\beta$-phenäthyl)-spiro[dibenz(b,f)thiepin-10,4'-piperidin] erhält.

Beispiel 24

Im Verlauf von 20 Minuten gibt man eine Lösung von 1,6 g 3-Phenoxypropylbromid in 10 ml Dimethylformamid portionsweise zu einem 80° C warmen Gemisch aus 2,1 g 2-Chlor-10,11-dihydro-11-oxospiro[dibenz(b,f)thiepin-10,4'-piperidin], der freien Base aus Beispiel 5, 2,1 g Natriumbicarbonat und 1,7 g Kaliumjodid in 15 ml Dimethylformamid. Nach beendeter Zugabe rührt man 16 Stunden bei 80—85° C. Danach wird im Eisbad abgekühlt und anschließend mit 75 ml Wasser verdünnt. Das verdünnte Gemisch extrahiert man mit 40 ml Äther und läßt beide Phasen (die organische und die wäßrige) durch Filterpapier laufen. Die filtrierte wäßrige Phase wird dreimal mit je 40 ml Äther extrahiert, und die vereinigten Ätherextrakte werden nacheinander zweimal mit je 40 ml Wasser und einmal mit 20 ml gesättigter Kochsalzlösung gewaschen und zur Trockne gebracht. Der Rückstand wird gereinigt, indem man ihn mit Äther als Eluiermittel durch eine Aluminiumoxydsäule laufen läßt, wobei man 2-Chlor-10,11-dihydro-11-oxo-1'-(3-phenoxypropyl)-spiro[dibenz(b,f)thiepin-10,4'-piperidin] erhält.

Beispiel 25

Ein Gemisch aus 1,8 g 4-Chlor-p-fluorbutyrophenon-äthylenketal und 10 ml Dimethylformamid gibt man im Verlauf von 30 Minuten portionsweise zu einem 85° C warmen Gemisch aus 2,1 g 2-Chlor-10,11-dihydro-11-oxospiro[dibenz(b,f)thiepin-10,4'-piperidin], der freien Base aus Beispiel 5, 1,7 g Kaliumjodid und 2,1 g Natriumbicarbonat in 15 ml Dimethylformamid. Nach beendeter Zugabe rührt man 16 Stunden bei 80—85° C und läßt dann 48 Stunden stehen. Danach wird mit 75 ml Wasser verdünnt und das verdünnte Gemisch dreimal mit je 40 ml Äther extrahiert. Die vereinigten Ätherextrakte wäscht man zweimal mit je 30 ml Wasser und dampft dann am Rotationsverdampfer ein, was einen glasigen Rückstand liefert. Dieser wird in einem Gemisch aus 55 ml Äthylalkohol und 21 ml 3n-Salzsäure gelöst. Die saure Lösung rührt man 19 Stunden bei Raumtemperatur. Das Gemisch wird

unter gutem Rühren im Eisbad gekühlt und dann mit 58%igem Ammoniumhydroxyd auf pH 9 basisch gemacht. Das basische Gemisch extrahiert man dreimal mit je 50 ml Äther, wäscht die vereinigten Ätherextrakte nacheinander zweimal mit je 30 ml Wasser und einmal mit 20 ml gesättigter Kochsalzlösung, trocknet und dampft zur Trockne ein. Der Rückstand wird gereinigt, indem man ihn mit Äther als Eluiermittel durch eine Aluminiumoxydsäule laufen läßt. Das gereinigte Produkt wird in Äther gelöst und als sein Bromwasserstoffsäuresalze, 2-Chlor-1'-[3-(4-fluorbenzoyl)-propyl]-10,11-dihydro-11-oxospiro[dibenz(b,f)thiepin-10,4'-piperidin]-hydrobromid, gefällt.

Beispiel 26

a) Man hält ein Gemisch aus 147 g Jodbenzoesäure und 45,5 g Kaliumcarbonat in 57 ml Nitrobenzol 40 Minuten unter Rühren bei 160°C. Danach werden weitere 46,5 g Kaliumcarbonat und anschließend 73,1 g 4-Fluorthiophenol, nochmals 46,5 g Kaliumcarbonat und 0,35 g Kupferpulver zugesetzt. Das Reaktionsgemisch wird 45 Minuten in einem Bad bei 160°C gerührt. Der entstandene Feststoff wird isoliert und dann auf 0°C gekühlt, bevor man ihn mit 100 ml Wasser und 220 ml 6n-Salzsäure vermischt. Dann verdünnt man das wäßrige Gemisch auf 1 Liter Gesamtvolumen und rührt dann mit 450 ml Chloroform. Der dabei verbleibende weiße Feststoff wird isoliert und nacheinander mit Chloroform und Wasser gewaschen. Der Feststoff wird in heißem Aceton aufgelöst und die Acetonlösung zuerst filtriert und dann abgekühlt, was ein weißes kristallines Produkt liefert. Das Produkt kristallisiert man aus Aceton um und überführt es dann mit Kaliumcarbonat in ein Salz. Das Salz wird in Wasser gelöst und die wäßrige Lösung mit 1n-Salzsäure angesäuert, wobei man 2-(4-Fluorphenylthio)-benzoesäure erhält.

b) Unter Stickstoff bei Raumtemperatur tropft man 36,1 ml 70%iges Natrium-bis-(2-methoxyäthoxy)-aluminiumhydrid in Benzol im Verlauf einer Stunde unter Rühren zu einem Gemisch aus 15 g 2-(4-Fluorphenylthio)-benzoesäure und 150 ml Benzol. Nach beendeter Zugabe rührt man noch 30 Minuten weiter und läßt das Reaktionsgemisch dann 4 Tage lang stehen. Danach kühlt man auf 0°C ab und versetzt dann unter Rühren mit 100 ml 10%iger Natronlauge. Nach dieser Zugabe scheiden sich die Schichten, und die wäßrige Schicht wird zweimal mit je 100 ml Benzol extrahiert. Die vereinigten Benzollösungen wäscht man nacheinander zweimal mit je 100 ml Wasser und einmal mit 60 ml gesättigter Kochsalzlösung und bringt zur Trockne, wobei man 2-(4-Fluorphenylthio)-benzylalkohol erhält.

c) Unter Rühren bei Raumtemperatur tropft man 8,5 g Thionylchlorid im Verlauf von 10 Minuten zu einem Gemisch aus 13,0 g 2-(4-Fluorphenylthio)-benzylalkohol und 80 ml Benzol. Nach beendeter Zugabe rührt man 16 Stunden und gießt das Reaktionsgemisch dann vorsichtig auf 350 ml zerkleinertes Eis, das 8 ml Natriumbicarbonat enthält. Man rührt, bis das Eis schmilzt. Die Schichten scheiden sich, und die Benzolschicht wird nacheinander zweimal mit je 40 ml halbgesättigter Natriumbicarbonatlösung, zweimal mit je 40 ml Wasser und einmal mit 25 ml gesättigter Kochsalzlösung gewaschen, getrocknet und am Rotationsverdampfer eingedampft, wobei man 2-(4-Fluorphenylthio)-benzylchlorid erhält.

d) Im Verlauf von 30 Minuten gibt man 12,0 g 2-(4-Fluorphenylthio)-benzylchlorid portionsweise zu einem Gemisch aus 2,9 g Natriumcyanid und 120 ml Dimethylsulfoxyd. Nach beendeter Zugabe rührt man 26 Stunden und gießt dann auf 150—200 ml zerkleinertes Eis. Man extrahiert viermal mit je 70 ml Äther und wäscht die vereinigten Ätherextrakte nacheinander viermal mit je 50 ml Wasser und einmal mit 25 ml gesättigter Kochsalzlösung und bringt zur Trockne, wobei man 2-(4-Fluorphenylthio)-benzylcyanid erhält.

e) Unter Stickstoff gibt man 2,3 g 99%iges Natriumhydrid langsam zu einem Gemisch aus 4,3 g 2-(4-Fluorphenylthio)-benzylcyanid und 30 ml Dimethylsulfoxyd. Nach beendeter Zugabe rührt man fünf Minuten weiter und gibt dann im Verlauf von 40 Minuten ein Gemisch aus 3,9 g 95%igem Mechloräthaminhydrochlorid und 20 ml Dimethylsulfoxyd portionsweise dazu. Nach dieser Zugabe rührt man eine Stunde in einem Heißwasserbad bei 70°C und läßt dann 16 Stunden bei Raumtemperatur stehen. Das abgekühlte Reaktionsgemisch gießt man auf 150 ml zerkleinertes Eis und extrahiert das verdünnte Gemisch dreimal mit je 60 ml Äther. Die vereinigten Ätherextrakte werden nacheinander mit 25 ml gesättigter Kochsalzlösung gewaschen und zur Trockne gebracht, wobei man 4-Cyan-4-[2-(4-fluorphenylthio)-phenyl]-1-methylpiperidin erhält.

f) Man rührt ein Gemisch aus 4,4 g 4-Cyan-4-[2-(4-fluorphenylthio)-phenyl]-1-methylpiperidin und 95 ml 48%iger Bromwasserstoffsäure 16 Stunden unter Rückfluß. Danach verdünnt man mit 300 ml Wasser und dampft bei 80°C am Rotationsverdampfer ein. Der Rückstand wird mit 300 ml Wasser umgeschwenkt und durch Erhöhung des pH auf 9 mit konzentriertem Ammoniumhydroxyd aufgelöst. Die Lösung läßt man 96 Stunden stehen. Der pH wird langsam auf 4 erniedrigt und dann mit konzentriertem Ammoniumhydroxyd wieder auf 9 gebracht. Das Volumen wird durch Rotationsverdampfung bei 80°C verringert, bis ein Niederschlag in Erscheinung tritt. Anschließend wird das Reaktionsgemisch gekühlt und der Niederschlag durch Filtrieren gesammelt und dann mit Wasser gewaschen. Nacheinander wird der Niederschlag mit Aceton gewaschen, mit 1 ml mit 20 ml Wasser verdünnter 48%iger Bromwasserstoffsäure behandelt und auf Bio-Rad A 650 W-X8-Kationenaustauscherharz aufgebracht und mit 5,8—8,7%igem Ammoniumhydroxyd eluiert, wobei man 4-[2-(4-Fluorphenylthio)-phenyl]-1-methylpiperidin-4-carbonsäure erhält.

g) Man rührt ein Gemisch aus 2,5 g 4-[2-(4-fluorphenylthio)-phenyl]-1-methylpiperidin-4-carbonsäure und 24 ml Polyphosphorsäure 2 Stunden unter Stickstoff in einem Bad bei 100—115°C. Danach kühlt man im Esibad und verdünnt dann mit 50 ml Wasser und 15 ml Eis. Das gekühlte verdünnte Gemisch wird mit 58%igem Ammoniumhydroxyd auf pH 9—10 basisch gemacht und dreimal mit je 50 ml Äther/Methylenchlorid (2 : 1) extrahiert. Man vereinigt die Extrakte und wäscht nacheinander mit 75 ml 10%iger Natronlauge, 75 ml Wasser und 20 ml gesättigter Kochsalzlösung und trocknet dann. Die getrocknete Lösung wird zur Trockne eingeengt, wobei ein Öl verbleibt, welches man über eine Silikagelsäule chromatographiert. Beim Eluieren mit Methylalkohol/Methylenchlorid (1 : 9) erhält man ein reines Produkt, das in Äther in sein Bromwasserstoffsäuresalz, 2-Fluor-10,11-dihydro-1'-methyl-11-oxospiro[dibenz(b,f)-thiepin-10,4'-piperidin]-hydrobromid, überführt wird.

Beispiel 27

Man gibt ein Gemisch aus 3,0 g 2-Fluor-10,11-dihydro-1'-methyl-11-oxospiro[dibenz(b,f)thiepin-10,4'-piperidin], der freien Base aus Beispiel 26, und 10 ml Methylenchlorid portionsweise zu einem Gemisch aus 1,3 g Bromcyan und 6,4 g Kaliumcarbonat in 17 ml Methylenchlorid. Nach beendeter Zugabe rührt man 2 Stunden unter Rückfluß und läßt dann auf Raumtemperatur abkühlen. Anschließend wird das Gemisch filtriert und dann zur Trockne eingedampft, und der Rückstand wird in Methylalkohol aufgenommen. Die alkoholische Lösung wird zum Rückfluß erhitzt und mit Aceton und Äther versetzt, was einen Niederschlag erzeugt. Man filtriert und dampft die überstehende Flüssigkeit ein. Der Rückstand wird über eine Silikagelsäule chromatographiert und mit Äther/Methylenchlorid (1 : 1) eluiert, wobei man 1'-Cyan-2-fluor-10,11-dihydro-11-oxospiro[dibenz(b,f)thiepin-10,4'-piperidin] erhält.

Beispiel 28

Man rührt ein Gemisch aus 1,8 g 1'-Cyan-2-fluor-10,11-dihydro-11-oxospiro[dibenz(b,f)thiepin-10,4'-piperidin], Beispiel 27, 11 ml Essigsäure und 21 ml 3n-Salzsäure 20 Stunden in einem Bad bei 125—130°C. Anschließend wird mit 25 ml Wasser verdünnt und am Rotationsverdampfer zur Trockne eingedampft. Der Rückstand wird in 25 ml Wasser aufgenommen und das Wasser am Rotationsverdampfer entfernt. Den Rückstand nimmt man in 25 ml Wasser auf und kühlt das wäßrige Gemisch auf 0°C. Der Niederschlag wird durch Filtrieren gewonnen und dann nacheinander mit Wasser und Äther gewaschen, bevor er getrocknet wird. Das Produkt wird mehrmals aus Methylalkohol/Aceton/Äther umkristallisiert, wobei man 2-Fluor-10,11-dihydro-11-oxospiro[dibenz(b,f)thiepin-10,4'-piperidin]-hydrochlorid erhält.

Beispiel 29

a) Im Verlauf von 15 Minuten gibt man ein Gemisch aus 19,8 g 4-Cyan-4-[2-(4-fluorphenylthio)-phenyl]-1-methylpiperidin, Beispiel 26(e), und 200 ml Chloroform portionsweise zu einem Gemisch aus 33,4 g 97%igem Bromcyan und 130 g Kaliumcarbonat in 250 ml Chloroform. Nach beendeter Zugabe wird das Gemisch nacheinander 16 Stunden zum Rückfluß erhitzt, abgekühlt, filtriert, dreimal mit je 300 ml Wasser und einmal mit 50 ml gesättigter Kochsalzlösung gewaschen und zur Trockne gebracht.

Der so erhaltene Rückstand wird in Benzol aufgelöst, mit Aktivkohle entfärbt und dann über Celite filtriert. Das Lösungsvolumen wird verringert, und es wird wieder mit Hexan verdünnt, wobei man 1,4-Dicyan-4-[2-(4-fluorphenylthio)-phenyl]-piperidin erhält.

b) Man rührt ein Gemisch aus 12,0 g 1,4-Dicyan-4-[2-(4-fluorphenylthio)-phenyl]-piperidin, Beispiel 29a), und 240 ml 48%iger Bromwasserstoffsäure 40 Stunden in einem Bad bei 150°C, versetzt dann mit 120 ml Eisessig und rührt noch 30 Stunden bei derselben Temperatur weiter. Danach wird die Lösung 64 Stunden stehengelassen und der Überschuß Säure dann abdestilliert. Der Rückstand wird in 50 ml Wasser aufgenommen, und das wäßrige Gemisch wird am Rotationsverdampfer eingedampft. Der Rückstand wird in 250 ml Methylakohol aufgelöst und die alkoholische Lösung mit Aktivkohle aufgekocht, wobei man 4-[2-(4-Fluorphenylthio)-phenyl]-piperidin-4-carbonsäurehydrobromid erhält.

c) Man rührt ein Gemisch aus 0,7 g 4-[2-(4-Fluorphenylthio)-phenyl]-piperidin-4-carbonsäure und 0,83 ml Essigsäureanhydrid in 4,2 ml Pyridin 4 Stunden unter Rückfluß. Anschließend entfernt man überschüssiges Pyridin am Rotationsverdampfer und nimmt den Rückstand in 10 ml Wasser auf, worin er mit 10 ml 1n-Salzsäure behandelt wird. Das saure Gemisch extrahiert man dreimal mit je 20 ml Äther/Benzol (1 : 1) und wäscht die vereinigten Extrakte nacheinander zweimal mit je 40 ml Wasser und einmal mit 15 ml gesättigter Kochsalzlösung und bringt zur Trockne. Der erhaltene Rückstand wird aus Aceton/Äther (1 : 4) umkristallisiert, wobei man 1-Acetyl-4-[2-(4-fluorphenylthio)-phenyl]-piperidin-4-carbonsäure erhält.

d) Man versetzt ein Gemisch aus 0,3 g 1-Acetyl-4-[2-(4-fluorphenylthio)-phenyl]-piperidin-4-carbonsäure und 3 ml Methylenchlorid mit 0,13 ml frisch destilliertem Thionylchlorid.

Das Gemisch rührt man 155 Minuten und verdünnt dann mit 7,5 ml Methylenchlorid. Die Reaktionslösung wird tropfenweise zu 0,17 g Aluminiumchlorid gegeben. Anschließend rührt man 15 Minuten und erhitzt dann drei Stunden zum Rückfluß. Das Gemisch wird unter Rückfluß 72 Stunden gerührt und dann nacheinander mit Eis, 25 ml Wasser und 25 ml Chloroform versetzt. Nach Umschütteln scheiden sich die Schichten. Die wäßrige Schicht wird zweimal mit je 25 ml Chloroform extrahiert, und die vereinigten Chloroformlösungen werden nacheinander einmal mit 20 ml 10%iger Natronlauge, zweimal mit je 30 ml Wasser und 1 ml gesättigter Kochsalzlösung gewaschen, getrocknet und eingedampft. Der Rückstand wird auf 20 × 20 cm großen präparativen Dickschichtsilikagelplatten mit Aceton/Äther/Methylenchlorid (1 : 1 : 1) chromatographiert. Das gereinigte Produkt wird in Benzol gelöst und mit Hexan verdünnt, und nach Entfernung des Lösungsmittels erhält man 1′-Acetyl-2-fluor-10,11-dihydro-11-oxospiro[dibenz(b,f)thiopin-10,4′-piperidin].

Weitere erfindungsgemäße Verbindungen lassen sich im Einklang mit den Arbeitsweisen der obenstehenden Beispiele herstellen.

**Patentansprüche für die Vertragsstaaten: BE, CH, DE, FR, GB, IT, NL, SE**

1. Verbindungen der Formel

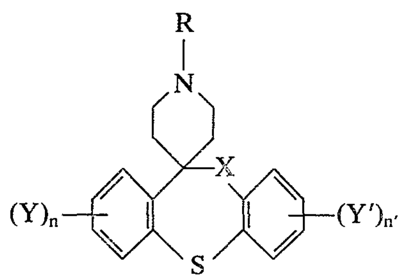

oder deren pharmazeutisch unbedenkliche Säureadditionssalze, worin R für Wasserstoff, $C_1-C_6$-Alkyl, $C_1-C_6$-Hydroxyalkyl, $C_3-C_7$-Cycloalkyl-$C_1-C_6$-alkyl, Phenylalkyl, Phenoxyalkyl oder Benzoylalkyl, worin die Alkylgruppe jeweils 1—6 C-Atome besitzt und der Phenylkern jeweils durch Nitro, Amino, Chlor, Fluor, Brom, Methoxy, $C_1-C_6$-Alkyl oder Trifluormethyl einfach oder mehrfach substituiert sein kann, Cyan oder ein Äthylenglykolketal der Formel

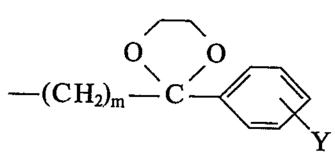

$C_2-C_6$-Alkanoyl oder einen Rest der Formel

$$-CH_2-R^1$$

mit $R^1$ = $C_2-C_5$-Alkenyl oder $C_2-C_5$-Alkinyl steht, X

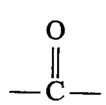

darstellt, wenn R die oben angegebene Bedeutung hat, oder

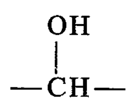

darstellt, wenn R für $C_1-C_6$-Alkyl oder Wasserstoff steht, Y und Y′ gleich oder verschieden sind und je Wasserstoff, Chlor, Fluor, Brom, Methoxy, Methylthio oder Trifluormethyl bedeuten können, n und n′ gleich oder verschieden sind und je eine ganze Zahl von 1 bis einschließlich 2 darstellen können und m eine ganze Zahl von 1 bis einschließlich 4 ist.

2. Verbindungen nach Anspruch 1, dadurch gekennzeichnet, daß Y für Wasserstoff und Y' für Wasserstoff, Chlor oder Fluor stehen und n' 1 ist.

3. Verbindungen nach Anspruch 1, dadurch gekennzeichnet, daß X für

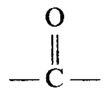

steht.

4. Verbindungen nach Anspruch 3, dadurch gekennzeichnet, daß R für Wasserstoff, $C_1-C_6$ Alkyl, $C_3-C_7$-Cycloalkyl-$C_1-C_6$-alkyl, gegebenenfalls substituiertes Phen-$C_1-C_6$-alkyl oderBenzoyl-$C_1-C_6$-alkyl oder einen Rest der Formel $-CH_2-R^1$ mit $R^1 = C_2-C_5$-Alkenyl steht.

5. Verbindungen nach Anspruch 4, dadurch gekennzeichnet, daß Y für Wasserstoff und Y' für Wasserstoff, Chlor oder Fluor stehen und n' 1 ist.

6. Verbindungen nach Anspruch 1, dadurch gekennzeichnet, daß R für Wasserstoff, Alkyl mit 1 bis 3 Kohlenstoffatomen, den Allyl- oder 2-Propinylrest, Hydroxyäthyl, Cycloalkylalkyl mit 4 bis 8 Kohlenstoffatomen, gegebenenfalls substituiertes Phenoxyalkyl oder gegebenenfalls substituiertes Phenylalkyl mit 1 bis einschließlich 3 Kohlenstoffatomen in der Alkylgruppe, gegebenenfalls substituiertes Benzoylalkyl mit 1 bis einschließlich 3 Kohlenstoffatomen im Alkylteil, Cyan oder ein Äthylenglykolketal der Formel

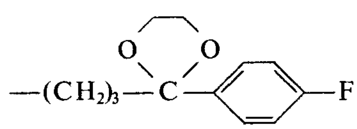

steht.

7. 2-Chlor-10,11-dihydro-1'-methyl-11-oxospiro[dibenz(b,f)-thiepin-10,4'-poperidin], oder ein pharmazeutisch unbedenkliches Säureadditionssalz davon.

8. 2-Chlor-10,11-dihydro-11-oxospiro[dibenz(b,f)thiepin-10,4'-piperidin], oder ein pharmazeutisch unbedenkliches Säureadditionssalz davon.

9. 2-Chlor-10,11-dihydro-1'-äthyl-11-oxospiro[dibenz(b,f)thiepin-10,4'-piperidin], oder ein pharmazeutisch unbedenkliches Säureadditionssalz davon.

10. 1'-[3-(4-Fluorbenzoyl)-propyl]-10,11-dihydro-11-oxospiro[dibenz(b,f)thiepin-10,4'-piperidin], oder ein pharmazeutisch unbedenkliches Säureadditionssalz davon.

11. 10,11-Dihydro-11-oxo-1'-(2-propinyl)-spiro[dibenz(b,f)thiepin-10,4'-piperidin], oder ein pharmazeutisch unbedenkliches Säureadditionssalz davon.

12. 2-Chlor-1'-cyclopropylmethyl-10,11-dihydro-11-oxospiro[dibenz(b,f)thiepin-10,4'-piperidin], oder ein pharmazeutisch unbedenkliches Säureadditionssalz davon.

13. 2-Fluor-11,11-dihydro-11-oxospiro[dibenz(b,f)thiepin-10,4'-piperidin], oder ein pharmazeutisch unbedenkliches Säureadditionssalz davon.

14. 2-Fluor-10,11-dihydro-1'-methyl-11-oxospiro[dibenz(b,f)thiepin-10,4'-piperidin], oder ein pharmazeutisch unbedenkliches Säureadditionssalz davon.

15. Verbindungen der Formel I

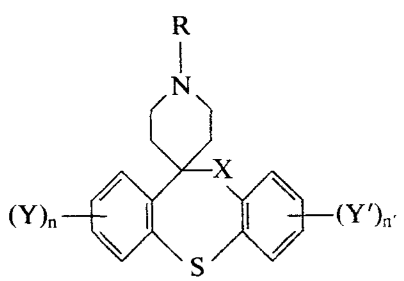

(I)

worin Y, Y', n und n' die zur Formel I in Anspruch 1 genannten Bedeutungen haben, X die Carbonylgruppe und R die Cyangruppe bedeutet, oder X die Gruppe

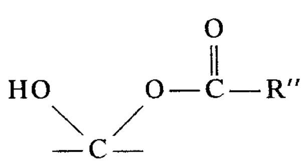

darstellt und R für

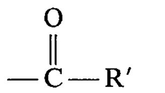 $CH_3$ oder CN

steht, wobei R' und R'' gleich oder verschieden sind und jeweils $C_1-C_6$-Alkyl bedeuten.

16. Verbindungen der Formel I gemäß Anspruch 1 zur Verwendung als Arzneimittel.

17. Arzneimittel, dadurch gekennzeichnet, daß es zwischen 0,5 und 70 Gew.-% einer Verbindung der Formel I gemäß Anspruch 1 und einen pharmazeutisch unbedenklichen Träger enthält.

**Patentanspruch für den Vertragsstaat: AT**

1. Verfahren zur Herstellung von Verbindungen der Formel I

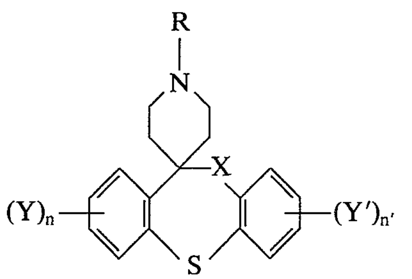 (I)

oder deren pharmazeutisch unbedenklichen Säureadditionssalzen; worin R für Wasserstoff, $C_1-C_6$-Alkyl, $C_1-C_6$-Hydroxyalkyl, $C_3-C_7$-Cycloalkyl-$C_1-C_6$-alkyl, Phenylalkyl, Phenoxyalkyl oder Benzoylalkyl, worin die Alkylgruppe jeweils 1—6 C-Atome besitzt und der Phenylkern jeweils durch Nitro, Amino, Chlor, Fluor, Brom, Methoxy, $C_1-C_6$-Alkyl oder Trifluormethyl einfach oder mehrfach substituiert sein kann, Cyan oder ein Äthylenglykolketal der Formel

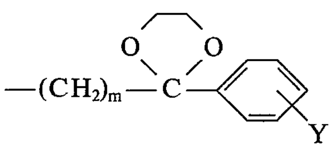

$C_2-C_6$-Alkanoyl oder einen Rest der Formel

$$-CH_2-R^1$$

mit $R^1 = C_2-C_5$-Alkenyl oder $C_2-C_5$-Alkinyl steht, X

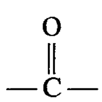

darstellt, wenn R die oben angegebene Bedeutung hat, oder

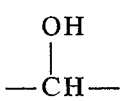

darstellt, wenn R für $C_1-C_6$-Alkyl oder Wasserstoff steht, Y und Y' gleich oder verschieden sind und je Wasserstoff, Chlor, Fluor, Brom, Methoxy, Methylthio oder Trifluormethyl bedeuten können, n und n' gleich oder verschieden sind und je eine ganze Zahl von 1 bis einschließlich 2 darstellen können und m eine ganze Zahl von 1 bis einschließlich 4 ist, dadurch gekennzeichnet, daß man

a) eine Verbindung der Formel

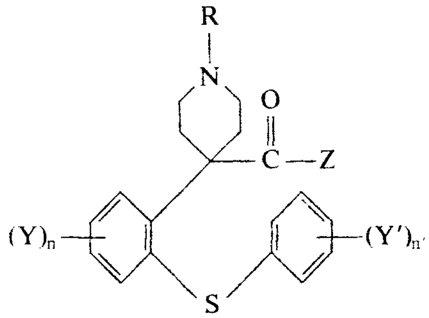

worin Y, Y', n und n' die zur Formel I genannten Bedeutungen haben, R für Methyl oder $C_2-C_6$-Alkanoyl und Z für ein Halogenatom oder eine Hydroxygruppe steht, cyclisiert,

b) gegebenenfalls die erhaltene Verbindung der Formel I, worin Y, Y', n und n' die oben genannten Bedeutungen haben, R $C_2-C_6$-Alkanoyl und X die Gruppe

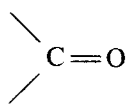

bedeutet, sauer hydrolysiert zu einer Verbindung der Formel I, worin R Wasserstoff bedeutet,

c) gegebenenfalls eine Verbindung der Formel I, worin R Methyl und X

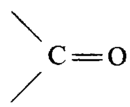

bedeutet, gemäß der ersten Stufe der von Braun-Reaktion mit Bromcyan umsetzt zu einer Verbindung der Formel I, worin R —CN bedeutet, und die erhaltene Verbindung der Formel I, worin R —CN bedeutet, gemäß der zweiten Stufe der von Braun-Reaktion hydrolysiert zu der entsprechenden Verbindung der Formel I, worin R Wasserstoff bedeutet,

d) gegebenenfalls eine Verbindung der Formel I, worin X die Gruppe C=O und R —CN, $CH_3$ oder $C_2-C_6$-Alkanoyl bedeutet, unter Friedel-Crafts-Bedingungen acyliert zu einer Verbindung der Formel I, worin X die Gruppe

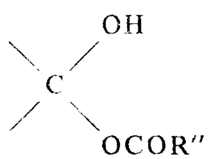

mit R'' = $C_1-C_6$-Alkyl bedeutet, und

e) gegebenenfalls eine Verbindung der Formel I, worin X

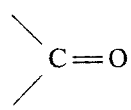

oder die Gruppe

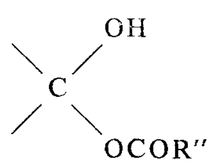

mit R'' = $C_1-C_6$-Alkyl bedeutet, und R für Wasserstoff, Methyl, CN oder $C_2-C_6$-Alkanoyl steht, reduziert zu einer Verbindung der Formel I, worin X die Gruppe

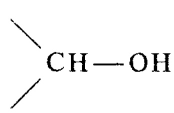

und R Wasserstoff oder $C_1-C_6$-Alkyl darstellt,

f) gegebenenfalls eine Verbindung der Formel I, worin R Wasserstoff und X

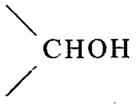 oder 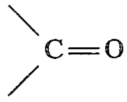

bedeutet, alkyliert oder acyliert zu einer Verbindung der Formel I, worin R $C_1-C_6$-Alkyl, $C_1-C_6$-Hydroxyalkyl, $C_3-C_7$-Cycloalkyl-$C_1-C_6$-alkyl, $C_2-C_6$-Alkanoyl, Phenyl-$C_1-C_6$-alkyl oder Phenoxy-$C_1-C_6$-alkyl oder Benzoyl-$C_1-C_6$-alkyl, worin der Phenylkern jeweils durch Nitro, Amino, Chlor, Fluor, Brom, Methoxy, $C_1-C_6$-Alkyl oder Trifluormethyl einfach oder mehrfach substituiert sein kann, oder einen Rest der Formel $-CH_2\ R^1$ mit $R^1 = C_2-C_5$-Alkenyl oder $C_2-C_5$-Alkinyl bedeutet,

g) gegebenenfalls eine Verbindung der Formel I worin X

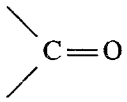

oder die Gruppe

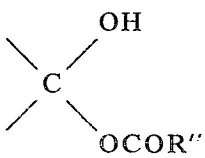

mit $R'' = C_1-C_6$-Alkyl und R Wasserstoff bedeutet, mit einem Chlorethylenglykolketal der Formel

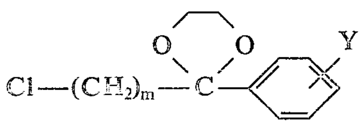

worin Y und m die oben genannten Bedeutungen haben, umsetzt zu einer Verbindung der Formel I, worin R die entsprechende Benzoylalkylethylenketalgruppe darstellt,

h) gegebenenfalls die erhaltene Verbindung sauer hydrolysiert zu einer Verbindung der Formel I, worin R die entsprechende Benzoylalkylgruppe darstellt,

i) gegebenenfalls eine Verbindung der Formel I, worin R Wasserstoff und X

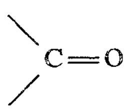 oder 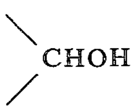

bedeutet, gemäß den Bedingungen der Eschweiler-Clarke-Reaktion umsetzt zu einer Verbindung der Formel I, worin X die genannten Bedeutungen hat und R Methyl darstellt,

j) und gegebenenfalls die erhaltenen Verbindungen der Formel I in ihre pharmazeutisch unbedenklichen Säureadditionssalze überführt.

**Claims for the Contracting states: BE, CH, DE, FR, GB, IT, NL, SE**

1. A compound of the formula

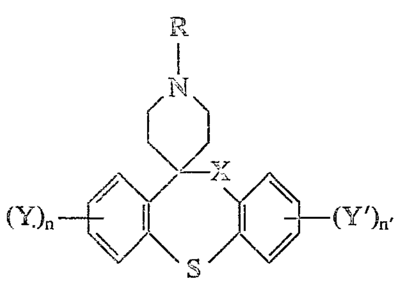

or a pharmaceutically acceptable acid addition salt thereof, in which R is hydrogen, $C_1-C_6$-alkyl,

$C_1-C_6$-hydroxyalkyl, $C_3-C_7$-cycloalkyl, $C_1-C_6$-alkyl, phenylalkyl, phenoxyalkyl or benzoylalkyl, wherein each alkyl group has 1–6 C-atoms and each phenyl radical may be one or more times substituted with nitro, amino, chlorine, fluorine, bromine, methoxy, $C_1-C_6$-alkyl or trifluoromethyl, or R is cyano, ethylene glycol ketal of the formula $C_2-C_6$-alcanoyl, or a radical of the

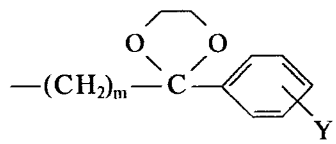

formula

$$-CH_2-R^1$$

with $R^1$ being $C_2$–$C_5$-alkenyl or $C_2$–$C_5$-alkinyl, X is

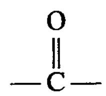

when R is as defined previously, or

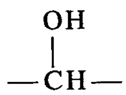

when R is $C_1-C_6$-alkyl or hydrogen, Y and Y′ are the same or different and each can be hydrogen, chlorine, fluorine, bromine, methoxy, methylthio or trifluoromethyl; n and n′ are the same or different and each can be an integer from 1 to 2, inclusive; and m is an integer from 1 to 4; inclusive.

2. A compound as defined in claim 1 in which Y is hydrogen, Y′ is hydrogen, chlorine or fluorine and n′ is 1.

3. A compound as defined in claim 1 in which X is

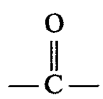

4. A compound as defined in claim 3 in which R is hydrogen, $C_1-C_6$-alkyl, $C_3-C_7$-cycloalkyl-$C_1-C_6$-alkyl, unsubstituted or substituted phenyl-$C_1-C_6$-alkyl or benzoyl-$C_1-C_6$-alkyl, or a radical of the formula $-CH_2-R^1$ with $R^1$ being $C_2-C_5$-alkenyl.

5. A compound as defined in claim 4 in which Y is hydrogen and Y′ is hydrogen, chlorine or fluorine and n′ is 1.

6. A compound as defined in claim 1 in which R is hydrogen, $C_1-C_3$-alkyl, allyl or 2-propinyl, hydroxyethyl, $C_4-C_8$-cycloalkylalkyl, unsubstituted or substituted phenoxyalkyl, phenylalkyl or benzoylalkyl in which the alkyl group has 1 to 3 inclusive, carbon atoms, cyano or ethylene glycol ketal of the formula

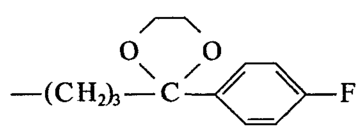

7. 2-Chloro-10,11-dihydro-1′-methyl-11-oxospiro[dibenz(b,f)thiepin-10,4′-piperidine] or a pharmaceutically acceptable acid addition salt thereof.

8. 2-Chloro-10,11-dihydro-11-oxospiro[dibenz(b,f)thiepin-10,4′-piperidine] or a pharmaceutically acceptable acid addition salt thereof.

9. 2-Chloro-10,11-1′-ethyl-dihydro-11-oxospiro[dibenz(b,f)thiepin-10,4′-piperidine] or a pharmacentically acceptable acid addition salt thereof.

10. 1′-[3-(4-fluorobenzoyl)propyl]-10,11-dihydro-11-oxospiro[dibenz(b,f)thiepin-10,4′-piperidine] or a pharmaceutically acceptable acid addition salt thereof.

11. 10,11-dihydro-11-oxo-1′-(2-propynyl)spiro[dibenz(b,f)thiepin-10,4′-piperidine] or a pharmaceutically acceptable acid addition salt thereof.

12. 2-Chloro-1′-cyclopropylmethyl-10,11,dihydro-11-oxospiro[dibenz(b,f)thiepin-10,4′-piperidine] or a pharmaceutically acceptable acid addition salt thereof.

13. 2-Fluoro-10,11-dihydro-11-oxospiro[dibenz(b,f)thiepin-10,4′-piperidine] or a pharmaceutically

acceptable acid addition salt thereof.

14. 2-Fluoro-10,11-dihydro-1′-methyl-11-oxospiro[dibenz(b,f)thiepin-10,4′-piperidine] or a pharmaceutically acceptable acid addition salt thereof.

15. A compound of the formula

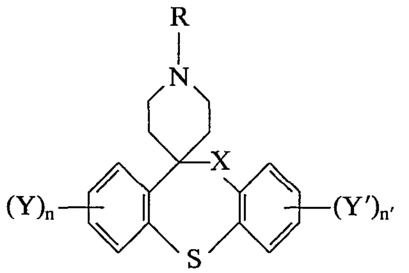

(I)

wherein Y, X′, n and n′ are as defined in formula I in claim 1, X is carbonyl and R is cyano, or X is the radical

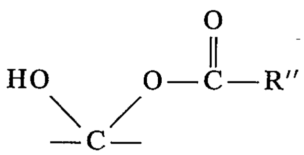

and R is the radical

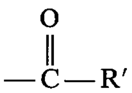

or is $CH_3$ or CN, R′ and R″ being the same or different and represent $C_1-C_6$-alkyl.

16. A compound of the formula I as defined in claim 1 for use as a medicament.

17. A pharmaceutical preparation which comprises 0,5 to 70% by weight of a compound of the formula I defined in claim 1 and a pharmaceutically acceptable carrier therefor.

**Claim for the Contracting state: AT**

A process for preparing a compound of the formula I

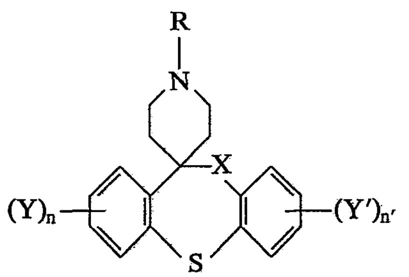

(I)

or a pharmaceutically acceptable acid addition salt thereof, in which R is hydrogen, $C_1-C_6$-alkyl, $C_1-C_6$-hydroxyalkyl, $C_3-C_7$-cycloalkyl-$C_1-C_6$-alkyl, phenylalkyl, phenoxyalkyl or benzoylalkyl, wherein each alkyl group has 1–6 C-atoms and each phenyl radical may be one or more times substituted with nitro, amino, chlorine, fluorine, bromine, methoxy, $C_1-C_6$-alkyl or trifluoromethyl, or R is cyano, ethylene glycol ketal of the formula $C_2-C_6$-alcanoyl, or a radical of the

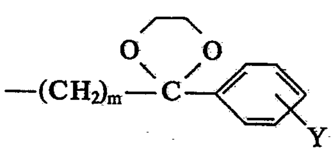

formula

$-CH_2-R^1$

with $R^1$ being $C_2$–$C_5$-alkenyl or $C_2$–$C_5$-alkinyl, X is

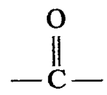

when R is as defined previously, or

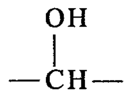

when R is $C_1-C_6$-alkyl or hydrogen, Y and Y′ are the same or different and each can be hydrogen, chlorine, fluorine, bromine, methoxy, methylthio or trifluoromethyl; n and n′ are the same or different and each can be an integer from 1 to 2, inclusive; and m is an integer from 1 to 4; inclusive, which comprises

a) cyclizing a compound of the formula

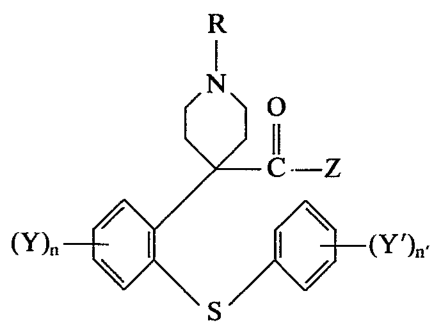

wherein Y, Y′, n and n′ is as defined above, R is methyl or $C_2-C_6$-alkanoyl and Z is hydroxy or halogen,

b) optionally subjecting a compound of the formula I wherein Y, Y′, n and n′ is as defined above and X is

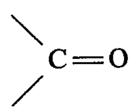

and R is $C_2-C_6$-alkanoyl to acid hydrolysis to provide a compound of the formula I wherein R is hydrogen,

c) optionally treating a compound of the formula I wherein R is methyl and X is

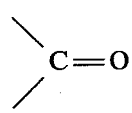

with cyanogen bromide in accordance with the first step of the von Braun reaction to provide a compound of the formula I wherein R is $-CN$, and optionally subjecting the compound obtained of the formula I wherein R is $-CN$ to hydrolysis according to the second step of the von Braun reaction to provide a compound of the formula I wherein R is hydrogen,

d) optionally acylating a compound of the formula I wherein X is

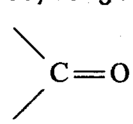

and R is $CH_3$, CN or $C_2-C_6$-alkanoyl under Friedel-Crafts conditions to provide a compound of the formula I wherein X is the group

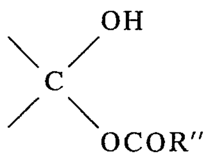

with R″ being $C_1-C_6$-alkyl and

e) optionally reducing a compound of the formula I wherein X is

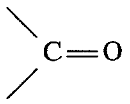

or the group

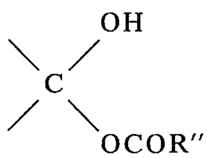

with R″ being $C_1-C_6$-alkyl, and R is hydrogen, Methyl, CN or $C_2-C_6$-alkanoyl, to provide a compound of the formula I wherein X is $>$CHOH and R is hydrogen or $C_1-C_6$-alkyl,

f) optionally alkylating or acylating a compound of the formula I wherein X is

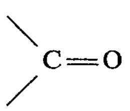 or 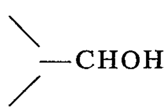

and R is hydrogen to provide a compound of the formula I wherein X is as previously defined and R is $C_1-C_6$-alkyl, $C_1-C_6$-hydroxyalkyl, $C_2-C_6$-alkanoyl, $C_3-C_7$-cycloalkyl-$C_1-C_6$-alkyl, phenyl-$C_1-C_6$-alkyl or phenoxy-$C_1-C_6$-alkyl or benzoyl-$C_1-C_6$-alkyl each phenyl radical of which may be substituted one or several times with nitro, amino, chlorine, bromine, fluorine, methoxy, trifluormethyl or $C_1-C_6$-alkyl, or a radical of the formula $-CH_2-R^1$ with $R^1$ being $C_2-C_5$-alkenyl or $C_2-C_5$-alkynyl,

g) optionally reacting a compound of the formula I wherein X is

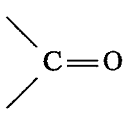

or the group

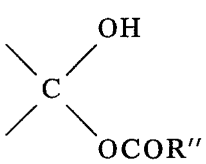

with R″ being $C_1-C_6$-alkyl and R is hydrogen, with a chloro ethylene glycol ketal of the formula

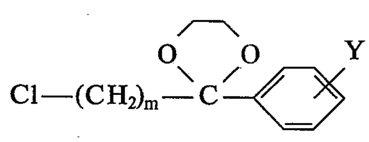

wherein Y and m are as defined above, to provide a compound of the formula I wherein R represents the corresponding benzoyl-alkylethylene ketal,

h) optionally subjecting the compound obtained to provide a compound of the formula I wherein R is the corresponding benzoylalkyl radical.

i) optionally treating a compound of the formula I wherein X is

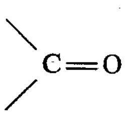 or 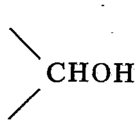

and R is hydrogen according to the conditions of the Eschweiler-Clarke reaction to provide a compound of the formula I wherein X is as previously defined and R is methyl,

j) and optionally transforming the compounds of formula I this obtained in their pharmaceutically acceptable acid addition salts.

**Revendications pour les Etats contractants: BE, CH, DE, FR, GB, IT, NL, SE**

1. Composés répondant à la formule

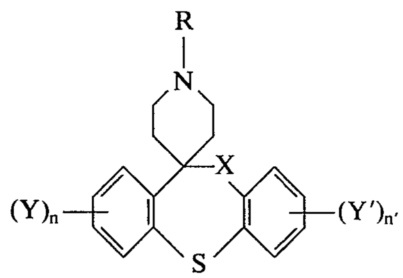

ou leurs sels d'addition avec des acides pharmaceutiquement acceptables, dans laquelle R désigne l'hydrogène, un groupe alcoyle en $C_1$ à $C_6$, un groupe hydroxyalcoyle en $C_1$ à $C_6$, un groupe cycloalcoyle en $C_3$ à $C_7$-alcoyle en $C_1$ à $C_6$, un groupe phénylalcoyle, phénoxyalcoyle, ou un groupe benzoylalcoyle dans lequel le groupe alcoyle possède à chaque fois 1 à 6 atomes de carbone et le noyau phényle peut être mono- ou polysubstitué par des groupes nitro, amino, chloro, fluoro, bromo, méthoxy, alcoyle en $C_1$ à $C_6$ ou trifluorométhyle, un groupe cyano ou un groupe acétal d'éthylèneglycol répondant à la formule

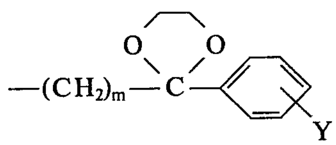

un groupe alcanoyle en $C_2$ à $C_6$ ou un radical répondant à la formule

$$-CH_2-R^1$$

avec $R^1$ = alcényle en $C_2$ à $C_5$ ou alcinyle en $C_2$ à $C_5$, X représente un groupe

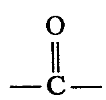

lorsque R a la signification indiquée ci-dessus, ou un groupe

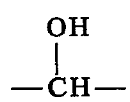

lorsque R désigne un groupe alcoyle en $C_1$ à $C_6$ ou l'hydrogène, Y et Y' sont identiques ou différents, et peuvent représenter chacun l'hydrogène, un groupe chloro, fluoro, bromo, méthoxy, méthylthio ou trifluorométhyle, n et n' sont identiques ou différents et peuvent représenter chacun un nombre entier de 1 à 2 compris, et m est un nombre entier de 1 à 4 compris.

2. Composés suivant la revendication 1, caractérisés en ce que Y désigne l'hydrogène, Y' l'hydrogène, un atome de chlore ou de fluor et n' est égal à 1.

3. Composés suivant la revendication 1, caractérisés en ce que X désigne

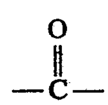

4. Composés suivant la revendication 3, caractérisés en ce que R désigne l'hydrogène, un groupe alcoyle en $C_1$ à $C_6$, cycloalcoyle en $C_3$ à $C_7$-alcoyle en $C_1$ à $C_6$, un groupe phénylalcoyle en $C_1$ à $C_6$ ou benzoylalcoyle en $C_1$ à $C_6$ éventuellement substitué ou un radical répondant à la formule $-CH_2-R^1$, avec $R^1$ = alcényle en $C_2$ à $C_5$.

5. Composés suivant la revendication 4, caractérisés en ce que Y représente l'hydrogène, et Y' l'hydrogène, un atome de chlore ou de fluor et n' est égal à 1.

6. Composés suivant la revendication 1, caractérisés en ce que R désigne l'hydrogène, un groupe alcoyle en $C_1$ à $C_3$, le radical allyle ou 2-propinyle, un radical hydroxyéthyle, cycloalcoyle en $C_4$ à $C_8$, un groupe phénoxyalcoyle éventuellement substitué ou un groupe phénylalcoyle éventuellement substitué ayant de 1 à 3 atomes de carbone dans le groupe alcoyle, un groupe benzoylalcoyle éventuellement substitué ayant de 1 à 3 atomes de carbone dans le groupe alcoyle, un groupe cyano ou un groupe acétal d'éthylèneglycol répondant à la formule

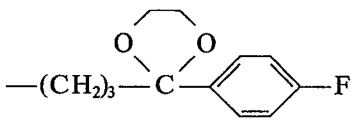

7. 2-chloro-10,11-dihydro-1'-méthyl-11-oxospiro[dibenzo(b,f)-thiépine-10,4'-pipéridine], ou un de ses sels d'addition avec des acides pharmaceutiquement acceptables.

8. 2-chloro-10,11-dihydro-11-oxospiro[dibenzo(b,f)thiépine-10,4'-pipéridine], ou un de ses sels d'addition avec des acides pharmaceutiquement acceptables.

9. 2-chloro-10,11-dihydro-1'-éthyl-11-oxospiro[dibenzo(b,f)-thiépine-10,4'-pipéridine], ou un de ses sels d'addition avec des acides pharmaceutiquement acceptables.

10. 1'-[3-(4-fluorobenzoyl)-propyl]-10,11-dihydro-11-oxospiro[dibenzo(b,f)thiépine-10,4'-pipéridine], ou un de ses sels d'addition avec des acides pharmaceutiquement acceptables.

11. 10,11-dihydro-11-oxo-1'-(2-propinyl)-spiro[dibenzo(b,f)thiépine-10,4'-pipéridine], ou un de ses sels d'addition avec des acides pharmaceutiquement acceptables.

12. 2-chloro-1'-cyclopropylméthyl-10,11-dihydro-11-oxospiro[dibenzo(b,f)thiépine-10,4'-pipéridine], ou un de ses sels d'addition avec des acides pharmaceutiquement acceptables.

13. 2-fluoro-11,11-dihydro-11-oxospiro[dibenzo(b,f)thiépine-10,4'-pipéridine], ou un de ses sels d'addition avec des acides pharmaceutiquement acceptables.

14. 2-fluoro-10,11-dihydro-1'-méthyl-11-oxospiro[dibenzo(b,f)thiépine-10,4'-pipéridine], ou un de ses sels d'addition avec des acides pharmaceutiquement acceptables.

15. Composés répondant à la formule I

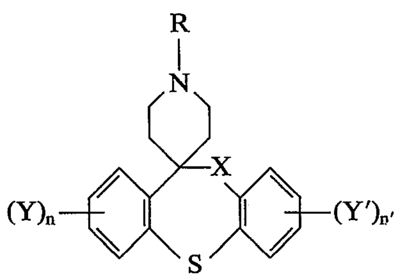

(I)

dans laquelle Y, Y', n et n' ont les significations indiquées à propos de la formule I dans la revendication 1, X désigne le groupe carbonyle et R le groupe cyano, à moins que X ne représente le groupe

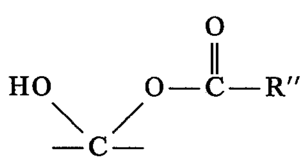

et R les groupes

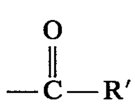 $CH_3$ ou CN

R' et R'' étant identiques ou différents et désignant chacun un groupe alcoyle en $C_1$ à $C_6$.

16. Composés répondant à la formule 1, suivant la revendication 1, destinés à être utilisés comme médicament.

17. Médicament, caractérisé en ce qu'il contient entre 0,5 et 70% en poids d'un composé répondant à la formule 1 suivant la revendication 1, et un support pharmaceutiquement acceptable.

**Revendication pour l'Etat contractant: AT**

Procédé de préparation de composés répondant à la formule I

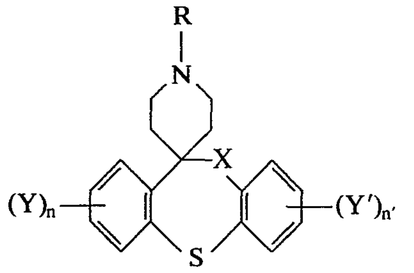

(I)

ou leurs sels d'addition avec des acides pharmaceutiquement acceptables, dans laquelle R désigne l'hydrogène, un groupe alcoyle en $C_1$ à $C_6$ un groupe hydroxyalcoyle en $C_1$ à $C_6$, un groupe cycloalcoyle en $C_3$ à $C_7$-alcoyle en $C_1$ à $C_6$, un groupe phénylalcoyle, phénoxyalcoyle, ou un groupe benzoylalcoyle dans lequel le groupe alcoyle possède à chaque fois 1 à 6 atomes de carbone et le noyau phényle peut être mono- ou polysubstitué par des groupes nitro, amino, chloro, fluoro, bromo, méthoxy, alcoyle en $C_1$ à $C_6$ ou trifluorométhyle, un groupe cyano ou un groupe acétal d'éthylèneglycol répondant à la formule

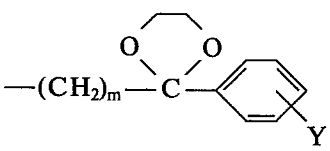

un groupe alcanoyle en $C_2$ à $C_6$ ou un radical répondant à la formule

$$-CH_2-R^1$$

avec $R^1$ = alcényle en $C_2$ à $C_5$ ou alcinyle en $C_2$ à $C_5$, X représente un groupe

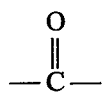

lorsque R a la signification indiquée ci-dessus, ou un groupe

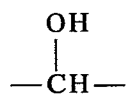

lorsque R désigne un groupe alcoyle en $C_1$ à $C_6$ ou l'hydrogene, Y et Y' sont identiques ou différents, et peuvent représenter chacun l'hydrogène, un groupe chloro, fluoro, bromo, méthoxy, méthylthio ou trifluorométhyle, n et n' sont identiques ou différents et peuvent représenter chacun un nombre entier de 1 à 2 compris, et m est un nombre entier de 1 à 4 compris, procédé caractérisé en ce

a) qu'on soumet à une cyclisation un composé répondant à la formule

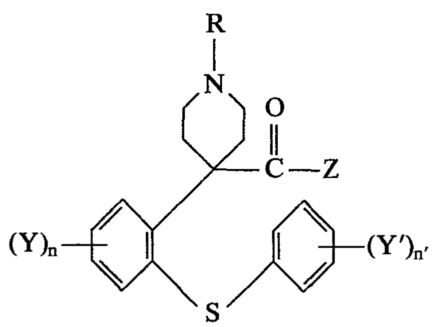

dans laquelle Y, Y', n et n' ont la signification indiquée dans la formule I, R désigne un méthyle un alcanoyle en $C_2$ à $C_6$ et Z désigne un atome d'halogène ou un groupe hydroxy,

b) le cas échéant, on soumet le composé obtenu répondant à la formule I, dans laquelle Y, Y', n et n' ont la signification indiquée ci-dessus, R désigne un alcanoyle en $C_2$ à $C_6$ et X désigne le groupe

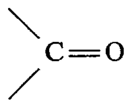

à une hydrolyse acide, ce qui fournit un composé répondant à la formule I, dans laquelle R désigne un atome d'hydrogène,

c) le cas échéant, on traite un composé répondant à la formule I, dans laquelle R désigne un méthyle et X désigne un groupe

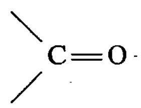

conformément au premier stade de la réaction de Braun, par un bromure de cyanogène, ce qui fournit un composé répondant à la formule I, dans laquelle R désigne un groupe $-CN$, et on soumet à une hydrolyse le composé obtenu répondant à la formule I dans laquelle R désigne un groupe $-CN$ conformément au second stade de la réaction de Braun, ce qui fournit un composé correspondant répondant à la formula I dans laquelle R désigne un atome d'hydrogène,

d) le cas échéant, on soumet à une acylation un composé répondant à la formule I dans laquelle X désigne le groupe $C=0$ et R désigne le groupe $-CN$, $CH_3$ ou un groupe alcanoyle en $C_2$ à $C_6$, dans des conditions de Friedel-Crafts, ce que fournit un composé répondant à le formule I dans laquelle X désigne le groupe

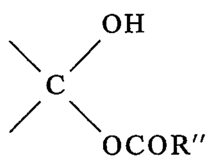

R'' étant un alcoyle en $C_1$ à $C_6$ et

e) le cas écheant, on réduit un composé répondant à la formule I dans laquelle X désigne un groupe

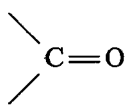

ou le groupe

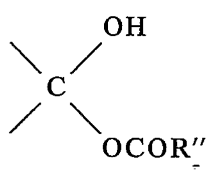

dans laquelle R'' désigne un alcoyle en $C_1$ à $C_6$, R désigne un atome d'hydrogène, un méthyle, un groupe CN ou un alcanoyle en $C_2$ à $C_6$, ce qui fournit un composé répondant à la formule I dans laquelle X désigne le groupe

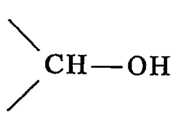

et R désigne un atome d'hydrogène ou un alcoyle en $C_1$ à $C_6$,

f) le cas échéant, on alcoyle ou on acyle un composé répondant à la formule I dans laquelle R désigne un atome d'hydrogène et X désigne un groupe

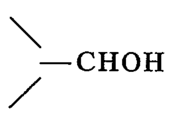

ou

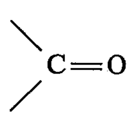

ce qui fournit un composé répondant à la formule I dans laquelle R désigne un groupe alcoyle en

$C_1$ à $C_6$, un groupe hydroxyalcoyle en $C_1$ à $C_6$, un groupe cycloalcoyle en $C_3$ à $C_7$-alcoyle en $C_1$ à $C_6$, un groupe alcanoyle en $C_2$ à $C_6$, un groupe phénylalcoyle ou phénoxyalcoyle ou un groupe benzoylalcoyle dans lesquels le groupe alcoyle possède à chaque fois de 1 à 6 atomes de carbone, le noyau phényle pouvant être porteur d'un ou de plusieurs radicaux pris dans l'ensemble constitué par un groupe nitro, amino, chloro, fluoro, bromo, méthoxy, alcoyle en $C_1$ à $C_6$ ou trifluorométhyle ou R désigne un radical de la formule $-CH_2R^1$ dans laquelle $R^1$ désigne un alcényle en $C_2$ à $C_5$ ou un alcinyle en $C_2$ à $C_5$,

g) le cas échéant, on fait réagir un composé répondant à la formule I dans laquelle X désigne un groupe

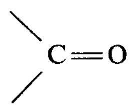

ou le groupe

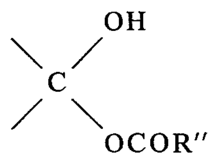

dans laquelle R'' désigne un alcoyle en $C_1$ à $C_6$ et R désigne un atome d'hydrogène, avec un acétal de chloroéthylèneglycol répondant à la formule

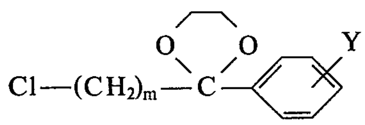

dans laquelle Y and m ont la signification donnée ci-dessus, ce qui fournit un composé répondant à la formule I dans laquelle R désigne le groupe éthylèneacétal de benzoylalcoyle,

h) le cas échéant, on soumet à une hydrolyse acide, le composé obtenu ce qui fournit un composé répondant à la formule I dans laquelle R désigne le groupe benzoylalcoyle correspondant,

i) le cas échéant on fait réagir un composé répondant à la formule I dans laquelle R désigne un atome d'hydrogène et X désigne un groupe

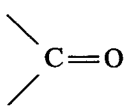
ou
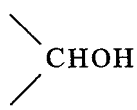

conformément aux conditions de la réaction d'Eschweiler-Clarke, ce qui fournit un composé répondant à la formule I dans laquelle X a la signification indiquée ci-dessus et R désigne un groupe méthyle et

j) le cas échéant, on transforme les composés obtenus répondant à la formule I, de manière habituelle, en leurs sels d'addition avec des acides pharmaceutiquement acceptables.